(19) Europäisches Patentamt
European Patent Office
Office européen des brevets

(11) **EP 3 745 945 B1**

(12) **EUROPEAN PATENT SPECIFICATION**

(45) Date of publication and mention
of the grant of the patent:
**22.05.2024 Bulletin 2024/21**

(21) Application number: **19702966.3**

(22) Date of filing: **28.01.2019**

(51) International Patent Classification (IPC):
***A61B 5/00*** (2006.01)

(52) Cooperative Patent Classification (CPC):
**A61B 5/0095**

(86) International application number:
**PCT/GB2019/050230**

(87) International publication number:
**WO 2019/145738 (01.08.2019 Gazette 2019/31)**

(54) **APPARATUS, METHODS AND COMPUTER READABLE MEDIUM FOR USE IN ULTRASOUND OPTICAL IMAGING**

VORRICHTUNG, VERFAHREN UND COMPUTERLESBARES MEDIUM ZUR VERWENDUNG BEI DER OPTISCHEN ULTRASCHALLABBILDUNG

APPAREIL, PROCÉDÉ ET SUPPORT LISIBLE PAR ORDINATEUR DESTINÉS À ÊTRE UTILISÉS DANS UNE IMAGERIE OPTIQUE ULTRASONORE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.01.2018 GB 201801450**
**09.11.2018 GB 201818317**

(43) Date of publication of application:
**09.12.2020 Bulletin 2020/50**

(73) Proprietor: **UCL Business Ltd**
**London WC1E 6BT (GB)**

(72) Inventors:
• **ZHANG, Edward**
**London W1T 4TP (GB)**
• **BEARD, Paul**
**London W1T 4TP (GB)**
• **HUYNH, Nam Trung**
**London W1T 4TP (GB)**

(74) Representative: **Barker Brettell LLP**
**100 Hagley Road**
**Edgbaston**
**Birmingham B16 8QQ (GB)**

(56) References cited:
JP-A- 2015 019 732     US-A- 5 590 090
US-A1- 2014 076 055     US-A1- 2016 095 520

• EDWARD ZHANG ET AL: "Backward-mode multiwavelength photoacoustic scanner using a planar Fabry-Perot polymer film ultrasound sensor for high-resolution three-dimensional imaging of biological tissues", APPLIED OPTICS, vol. 47, no. 4, 1 February 2008 (2008-02-01), page 561, XP055572931, WASHINGTON, DC; US ISSN: 0003-6935, DOI: 10.1364/AO.47.000561

**Description**

TECHNICAL FIELD

The present invention relates generally to methods and apparatuses for use in ultrasound optical imaging. The invention finds utility in performing photoacoustic tomography with respect to a sample that receives a pulse of excitation electromagnetic radiation and generates an acoustic field in response to said pulse.

BACKGROUND

Photoacoustic tomography is a noninvasive imaging technique that involves the irradiation of a surface of a tissue to be imaged with low energy nanosecond pulses of laser light, which is absorbed by subsurface anatomical features and leads to impulsive heating accompanied by rapid thermoelastic expansion and the subsequent generation of broadband (tens of megahertz) acoustic signal field of ultrasonic pulses. The ultrasonic pulses propagate to the surface where they are detected at multiple points using an appropriate ultrasonic signal transducer, from which a volumetric image of the internally absorbed optical energy distribution can be recreated, using tomographic techniques, revealing the internal tissue structure.

Photoacoustic tomography is advantageous as it has the potential to provide a high resolution imaging capability as the ultrasound field, which does not suffer significant internal scattering in the tissue, is used to reveal the energy absorption. Further, the application of light energy to the tissue provides a high contrast image as the light is selectively absorbed by the tissue structure and can be spectroscopically tuned to one or more desired absorption wavelengths to image different structural and functional aspects of the tissue physiology.

Particularly, photoacoustic tomography is envisaged to be useful in clinical environments wherein non-invasive imaging is preferable. It is particularly well suited to clinical dermatological applications that require visualising the structure and function (via the oxygenation status) of blood vessel networks in the skin. Among these are the assessment of burn depths, wound healing, pressure sores, plastic surgery procedures (grafts, flaps etc.) and characterising skin pathologies: for example, to provide a more accurate diagnosis and staging of tumours such as malignant melanomas and help plan their surgical excision. As well as clinical applications, the system is being evaluated as an investigative tool for undertaking basic research in the life sciences, for example studying the structure and oxygenation status of the microvasculature associated with superficial tumours in order to develop new cancer treatments. More generally, the instrument is intended for use in a variety of molecular imaging applications in cardiovascular biology, preclinical neurology and oncology.

Sensing the photoacoustic signal field using well established piezoelectric transducers is challenging, however, due to the difficulty of irradiating the tissue in the location of the piezo receiver due to obscuration by the transducer, and also because the broadband photoacoustic field requires the sensor elements to be extremely small - on the order of microns to tens of microns - which for a piezoelectric sensor significantly limits the sensitivity, making this approach impractical. Optical ultrasound detection provides a potentially viable alternative, whereby the photoacoustic field is sensed and revealed using optical means. In one optical ultrasound approach, a narrowband optical component having a transfer function responsive to an acoustic field applied to the optical component is used as a sensor to transduce the photoacoustic signal field. One such suitable optical component is a Fabry Perot etalon exhibiting narrowband reflection. By contacting a surface of the Fabry Perot etalon to the tissue surface, the etalon then has an optical path length of its cavity - and thus the location of the central wavelength of the Interferometer Transfer Function (ITF) of the etalon - modulated at points on the Fabry Perot etalon by the photoacoustic field locally incident at those points. This localised ITF modulation by the incident acoustic field can be interrogated by illuminating the etalon with a laser and monitoring the variation of the reflected optical power at locations across the surface as the central wavelength of the etalon ITF is locally modulated by the photoacoustic field incident on the surface. The acoustic field can thus be recovered from the modulated interrogation light signal, allowing an image of the target tissue to be constructed using tomographic techniques. This approach enables illumination of the tissue sample through the etalon, and also enables high sensitivity to be achieved.

Despite the potential of photoacoustic tomography using optical ultrasound detection, high quality, high resolution imaging results have typically only been achieved in a controlled research lab environment, with the time taken to reveal an image of a target typically taking a significant amount of time to capture and then process. This makes current photoacoustic tomography techniques impractical for clinical research and practice, and also for applications in life sciences research.

Efforts are ongoing to develop a photoacoustic imaging apparatus that is usable in a clinical and research setting and that can be operated to provide timely and accurate imaging results.

It is in this context that the presently disclosed subject matter has been devised.

Edward Zhang et. al., "Backward-mode multiwavelength photoacoustic scanner using a planar Fabry-Perot polymer film ultrasound sensor for high-resolution three-dimensional imaging of biological tissues", Applied Optics vol. 47, no. 4, 1 February 2008, page 561, describes a multiwavelength backward-mode planar photoacoustic scanner for 3D imaging of soft tissues to depths of several millimetres with a spatial resolution in the tens to hundreds of micrometres range. The system comprises a tuneable optical parametric oscillator laser system that provides nanosecond laser pulses between 600 and 1200 nm for generating the photoacoustic signals and an optical ultrasound mapping system based upon a Fabry-Perot polymer film sensor for detecting them.

US 2016/095520 A1 describes a method and apparatus are provided for performing photoacoustic tomography with respect to a sample that receives a pulse of excitation electromagnetic radiation and generates an acoustic field in response to said pulse.

BRIEF SUMMARY OF THE INVENTION

Viewed from one aspect, the present disclosure provides methods and apparatuses for performing ultrasound optical imaging with respect to a sample in which an acoustic field is generated in response to a stimulus. The apparatus comprises: a sensor head having an acoustically sensitive surface arranged as a reflective surface of a Fabry Perot interferometric cavity, wherein, when the acoustically sensitive surface is placed against the sample, in use, the acoustic field generated in response to said stimulus is incident upon said acoustically sensitive surface to modulate the optical path length in the cavity with a signal indicative of the acoustic field; a light source, optionally wavelength tuneable, for generating one or more interrogation beams of electromagnetic radiation; beam directing means to direct the one or more interrogation beams onto said acoustically sensitive surface; phase control means for controlling the phase difference between the optical fields reflected from the two mirrors of the Fabry Perot Interferometer, such as of the successive reflected light fields of the one or more interrogation beams in the cavity; detection means configured to receive and determine one or more values representative of the power of the reflected one or more interrogation beams; computer readable medium storing phase data representative of a set of phase difference values for controlling the phase control means, the phase data representing a determined bias phase, $\varphi_b$, for the sensor head, selected to be a phase difference of the light field in the cavity at which the power of the interrogation beam interferometrically reflected from the Fabry Perot cavity is in use modulated by the signal from the acoustic field incident on the acoustically sensitive surface at that location.

In embodiments, the beam directing means is configured to direct the one or more interrogation beams onto addressable locations (x,y) across said acoustically sensitive surface; the detection means is configured to receive and determine one or more values representative of the power of the reflected one or more interrogation beams from the addressable locations (x,y); and the phase data is stored as scanning phase data representative of a set of phase values for controlling the phase control means for addressable locations (x,y) of the sensor head, the scanning phase data relating: addressable locations (x,y) of the sensor head onto which the one or more interrogation beams can be directed in use; and a respective determined bias phase, $\varphi_b$, for the sensor head at given addressable locations (x,y).

The apparatus further comprises a controller configured to: access the scanning phase data stored in the computer readable medium; identify from the scanning phase data regions of grouped addressable locations (x,y) having a common bias phase, $\varphi_b$; for each identified region of the addressable locations (x,y): control the phase control means to control the phase difference of the light of the interrogation beam in the cavity to the bias phase, $\varphi_b$, of the region; control the beam directing means to sequentially interrogate addressable locations (x, y) of the group of locations making up the region; and receive, from detection means, values representative of the power of the reflected interrogation beam for each interrogated addressable location of the region; and form an image indicative of the signal modulated on the reflected one or more interrogation beams by the acoustic field incident on the acoustically sensitive surface at addressable locations (x, y) across the sensitive surface. In some embodiments, the controller may be configured to, for i=1 to n regions with common bias phases, sequentially perform the steps of: a) controlling the phase control means to control the phase difference of the light of the interrogation beam in the cavity to the current bias phase, $\varphi_{bi}$; b) control the beam directing means to sequentially interrogate addressable locations (x, y) of the group of locations making up the ith region, and c) receive, from the detection means, values representative of the power of the reflected interrogation beam for each interrogated addressable location of the ith region. In this way the phase difference is adjusted to match the first group, then the first group interrogated, then the phase difference adjusted to match the second group, then the second group interrogated, and so on.

Reference herein to phase difference, $\varphi$, or phase difference of light in the cavity is to be understood to mean the phase difference between the reflected optical fields of the light in the cavity, in particular, the phase difference between the optical fields reflected from the two mirrors of the Fabry Perot Interferometer, such as of the successive reflected light fields of the one or more interrogation beams in the cavity. The term "common bias phase" as used in this disclosure does not necessarily imply that the locations in question have an exactly identical bias phase. The term "common bias phase" is used herein to refer to regions with sufficiently similar bias phase for them to be grouped together. In some embodiments a common bias phase may refer to phase biases that are within a predetermined threshold % variance (e.g. 1%, or 2%), or with a predetermined absolute variance. In some embodiments the distribution of bias phase may alternatively be divided into a plurality of sub-ranges or bins (e.g. based on equal bias phase intervals, or with equal populations of locations), and each bin treated as having common bias phase. In certain embodiments, the interrogating step for each region interrogates (and/or obtains/retains values from) only the addressable locations of the group of locations making up the region. The beam directing means (in any aspect) may be configured to move a focussed interrogation beam across the acoustically sensitive surface to the addressable locations in order to interrogate the addressable locations.

Viewed from another aspect, the present disclosure provides a computer readable medium comprising instructions which when executed by one or more processors, cause the one or more processors to implement the method for performing ultrasound optical imaging as disclosed in above the above aspect.

While the ultrasound optical imaging technique disclosed herein is applicable to the sensing and imaging of any appropriate ultrasound acoustic field generated by a range of stimuli in a range of samples for a range of applications, in embodiments, the stimulus for generating the acoustic field is a pulse of excitation electromagnetic radiation and generates an acoustic field in the sample in response to said pulse. Thus, the acoustic field may be a photoacoustic field. The image may be constructed using tomographic techniques, for example, to recover a 3D image of the sample.

In the ultrasound optical imaging apparatuses disclosed herein, the phase control means can be any suitable control device or components or subcomponents or functionality of another, larger device that is configured to control the phase difference of the light of the one or more interrogation beams in the cavity. By controlling the phase difference of the light in the cavity, the apparatus can be 'tuned' across an interferometer transfer function of the resonant cavity, for example, to find a bias phase at which a sensitivity of the reflected power from the interferometer is highest to acoustic modulation.

In embodiments, the phase control means achieves this by tuning a wavelength of light produced by the wavelength tunable source. That is, the phase control means may control a tunable laser to change the wavelength of light in the cavity, for example to a bias wavelength. In this case, the scanning phase data may be stored as scanning wavelength data, relating: addressable locations (x,y) of the sensor head; and a respective determined bias wavelength, $\lambda_b$, for the wavelength source at given addressable locations (x,y). The bias wavelength is selected to be a tuned wavelength of the source at which the power of the interrogation beam interferometrically reflected from the Fabry Perot cavity is in use modulated by the signal from the acoustic field incident on the acoustically sensitive surface at that location. Indeed, embodiments in which the wavelength of the interrogation beam is tuned to tune the phase difference of light in the cavity will be focussed on in this application and described in detail in relation to the Figures. Indeed, references herein to 'scanning wavelength data' should be understood by the reader to be transposable to the broader phase-based tuning of the phase difference of light in the cavity.

Alternatively, or in addition, in embodiments, the phase control means may control the phase difference of the light in the cavity by controlling a thickness of the cavity. This may be achieved by, for example, controlling the interrogation light source to generate a heating pulse of controlled energy to heat the sensor head structure and locally heat the cavity to expand the cavity thickness to a bias thickness before interrogating the structure to measure the reflected power. Alternatively, a different laser beam, which may be of a different wavelength, and which could be pulsed or Continuous Wave, could be used to heat and control the cavity thickness. Alternatively, this may be achieved by the cavity being made of a material that expands and contracts responsive to an external applied influence such as an electromagnetic field that can be applied by the phase control means to bias the cavity thickness. In this case the scanning phase data may be stored as scanning thickness data, relating: addressable locations (x,y) of the sensor head; and a respective determined bias thickness, $t_b$, for the cavity at given addressable locations (x,y). The bias thickness is selected to be a tuned thickness of the cavity at which the power of the interrogation beam interferometrically reflected from the Fabry Perot cavity is in use modulated by the signal from the acoustic field incident on the acoustically sensitive surface at that location.

Alternatively, or in addition, in embodiments, the phase control means may control the phase difference of the light in the cavity by controlling a refractive index of the cavity. This may be achieved by the cavity or sensor head being made of a material that changes the refractive index of the cavity responsive to an external applied influence such as an electromagnetic field that can be applied by the phase control means to bias the cavity thickness. In this case the scanning phase data may be stored as scanning refractive index data, relating: addressable locations (x,y) of the sensor head; and a respective determined bias refractive index, $n_b$, for the cavity at given addressable locations (x,y). The bias refractive index is selected to be a tuned refractive index of the cavity at which the power of the interrogation beam interferometrically reflected from the Fabry Perot cavity is in use modulated by the signal from the acoustic field incident on the acoustically sensitive surface at that location.

That is, the controller and the phase control means may control one or more of the wavelength, thickness and refractive index to control the phase of light in the cavity.

In accordance with the above-disclosed aspects, the imaging of ultrasound acoustic fields, including photoacoustic fields, can be performed by using an ultrasound optical tomography technique to sense a acoustic signal field by scanning across a sensor head using an interrogation beam that permits reconstruction of an image of the tissue on a timescale fast enough to be practical and usable, for example, in a clinical and life sciences research setting. Where the phase control means tunes the wavelength of the light in the cavity, this is achieved by using a beam directing means to direct a focused beam of a tuneable laser to interrogate the sensor head across addressable locations. The beam directing means is controlled by a controller to interrogate the sensor head, for each determined bias wavelength of the sensor head, to scan all addressable locations in one or more grouped addressable locations defining regions of the sensor head having a common bias wavelength of interrogation. The regions are determined by using the stored scanning wavelength data which relates the addressable locations with a bias wavelength for those locations to build regions of the sensor head having a common bias wavelength. In this way, the sensor head is interrogated at all of those locations in those region(s) having a common bias wavelength before retuning the interrogation laser and proceeding to interrogate the next region(s) of the sensor head having that re-tuned wavelength. The interrogation proceeds in this way, region by region, then wavelength by wavelength, until all addressable locations on the sensor head have been interrogated.

In this way, the number of times the laser has to be tuned to a different wavelength to interrogate the sensor head is kept low. The present inventors have realised that this allows the sensor head interrogation to be performed very quickly, as the timescale for retuning a normal tuneable laser is relatively long, particularly when compared with the time taken to redirect the interrogation beam across different addressable locations on the sensor head. This 2-stage approach is advantageous because it reduces the time devoted to tuning the laser wavelength when recording the photoacoustic signals during the image acquisition stage, and so reduces the overall time required to take a scan of the sensor head by many orders of magnitude. Scanning and image reconstruction can thus be performed in a matter of seconds or less, rather than minutes or hours, and additionally dynamic operation is achievable giving a usable refresh rate.

Optionally, the sensor head is formed as a planar Fabry Perot etalon, wherein the etalon comprises the addressable locations (x,y) of the sensor head onto which the interrogation beam can be directed in use.

Optionally, the sensor head is formed to have an array of microresonator Fabry Perot interferometric cavities with plano-convex geometry, wherein individual microresonator cavities correspond to the addressable locations (x,y) of the sensor head onto which the interrogation beam can be directed in use.

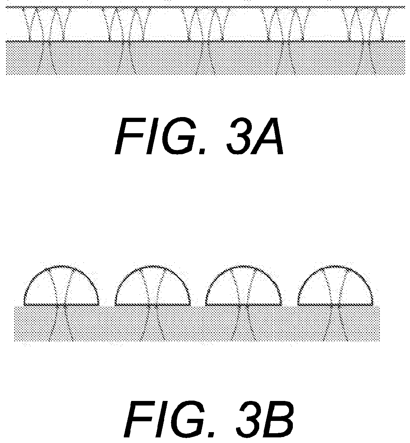

By providing a sensor head having addressable locations corresponding to plano-convex cavities (as shown in Figure 3B) offers significantly higher sensitivity and signal response since it avoids beam walk-off (i.e. the beam is contained at the addressable location), thereby allowing many more round trips within the cavity thus increasing its Q-factor.

Optionally, the determined bias phase, $\varphi_b$, for the addressable locations is selected to be substantially at an extrema of the derivative of an interferometer transfer function (ITF) for each addressable location generated during a tuning process of the apparatus, the ITF characterising the power of the reflected interrogation beam within a Free Spectral Range of the Fabry Perot Interferometer.

At this phase difference the sensitivity and linearity of the sensor response is a maximum.

Optionally, to adjust the determined bias phase, $\varphi_b$, to compensate for local or bulk changes in the optical path length in the cavity in use, such as due to variations in sensor head temperature and applied pressure since the tuning process, the controller is further configured to: in use, monitor the reflected power of the one or more reflected beams at one or more of the addressable locations (x,y) for bias tracking; for each of the one or more addressable locations (x,y) for bias tracking: compare the reflected power with a reflected power expected for the current bias phase $\varphi_b$ from the ITF generated during the tuning process for apparatus; and determine an adjustment $\delta\varphi_b$ to the current bias phase $\varphi_b$ needed to compensate for the change in the reflected power from the cavity; and, update the stored values of the determined bias phase, $\varphi_b$, in the scanning phase data for addressable locations (x,y) of the sensor head to a compensate bias phase value based on the determined adjustment $\delta\varphi_b$ to the currently stored bias phase $\varphi_b$ for the one or more addressable locations (x,y) for bias tracking.

In this way it is possible to track changes in the bias phase and maintain the sensor at optimum sensitivity in the presence of self-heating or external temperature and pressure changes.

In embodiments, the beam directing means may be an optical fibre or an optical fibre bundle. In this way the sensor head can be mounted on an optical fibre or fibre bundle.

Alternatively, the beam directing means may be a controllable beam directing means operable to direct the one or more interrogation beams onto addressable locations (x,y) across said acoustically sensitive surface. Optionally, the controller is configured to control the beam directing means to sequentially interrogate addressable locations (x, y) of the group of locations making up the region, the controller is further configured to: interrogate an addressable location at an extremity of the region, interrogate further addressable locations within the region, wherein each sequential location is determined by the nearest addressable location to the previously interrogated location.

The reason why minimising the step-size is important (both when scanning within a region and moving from one region to the next) is because the settling time for the beam directing means (typically comprised of mirror galvanometers) used to scan the laser beam scales with the step-size. Small steps are therefore enable rapid acquisition.

Optionally, the scanning phase data comprises a pre-tuning map, the pre-tuning map comprising the addressable locations mapped to the respective determined bias phase.

Optionally, the scanning phase data comprises a look-up table grouping the addressable locations by determined bias phase. Where phase difference is controlled by controlling the interrogation source wavelength, for example, this minimises the number of times the laser wavelength has to be adjusted. It is therefore significantly more efficient than x-y raster scanning and adjusting the laser wavelength at each x-y position.

Optionally, the scanning phase data comprises a scan-plan, the scan-plan comprising an ordered list of addressable locations, wherein addressable locations with the same bias phases are grouped together. Where phase difference is controlled by controlling the interrogation source wavelength, for example, the process of identifying the regions of common $\lambda_b$ and determining the optimum order of x,y positions (the "scan-plan") in order to scan most efficiently, both within a specific region of the same $\lambda_b$ and between regions of different $\lambda_b$ is called "grouping".

Thus the scanning phase or wavelength data can be stored in any appropriate way that allows region-by-region, phase-by-phase or wavelength-by-wavelength scanning in use. It can be stored as a simple tuning map for all addressable locations, and post-processed in the controller to group them into regions of common phase difference and generate a scan plan to control the beam directing means and the phase control means to carry out the tuning and scanning across the addressable locations region-by-region and phase -by- phase. Alternatively, the tuning map data may be pre-processed such that the scanning phase data may be stored as a grouped regions of common phase difference (e.g. in a look up table) for post processing into a scan plan, or as a scan plan itself.

Optionally, the apparatus further comprises beam splitting means to split the interrogation beam into a plurality of beams. Optionally, the plurality of beams are arranged in a line. The use of multiple beams, from a single tuneable wavelength source or even from multiple tuneable wavelength sources, further speeds up the scanning and interrogation process. Plural beam directing means may be provided to direct each of the multiple beams onto different addressable locations of the sensor head. The beam directing means may be arranged to scan the multiple beams in a line, allowing faster interrogation of grouped regions of the sensor head. Other geometries are possible for multiple beams, such as a 1D, 1.5D or 2D array and the beams may be arranged in any distribution within these geometries.

According to a non-claimed aspect, there is provided methods and apparatuses for generating a tuning map of a sensor head, the sensor head for use in performing ultrasound optical imaging with respect to a sample in which an acoustic field is generated in response to a stimulus. The apparatus comprises: a sensor head having an acoustically sensitive surface arranged as a reflective surface of a Fabry Perot interferometric cavity, wherein, when the acoustically sensitive surface is placed against the sample, in use, the acoustic field generated in response to said stimulus is incident upon said acoustically sensitive surface to modulate the optical path length in the cavity with a signal indicative of the acoustic field; a light source, optionally wavelength tuneable, arranged to generate one or more interrogation beams of electromagnetic radiation; beam directing means to direct the one or more interrogation beams onto addressable locations (x,y) across said acoustically sensitive surface; phase control means for controlling the phase difference of the light of the one or more interrogation beams in the cavity; detection means configured to receive and determine one or more values representative of the power of the reflected one or more interrogation beams from the addressable locations (x,y). The method is implemented by a controller configured to: operate the phase control means to tune the phase difference of light in the cavity to selected characterisation phases ($\varphi_{1...n}$) within the range of phases between two minima of the ITF (i.e. the Free Spectral Range where phase control is achieved by tuning the wavelength of the interrogation laser) of the Fabry Perot Interferometer; at each characterisation phase ($\varphi_{1...n}$), use the beam directing means to direct the one or more interrogation beams to each of at least a characterisation subset of the addressable locations (x,y) of the sensor head; detect, by the detections means, for at least the characterisation subset of the addressable locations (x,y) and each characterisation phase ($\varphi_{1...n}$), the power of the one or more reflected interrogation beams, to generate an interferometer transfer function $ITF_G$ (x,y). The controller may be further configured to: fit a template interferometer transfer function $ITF_T$ to each $ITF_G$ (x,y) to generate a fitted interferometer transfer function $ITF_F$(x,y) for each addressable location (x,y); determine, from the fitted interferometer transfer function $ITF_F$(x,y) for each addressable location (x,y), a respective bias phase, $\varphi_b$(x,y), for the light source at each addressable location (x,y), selected to be a tuned phase difference of the light in the cavity at which the power of the interrogation beam interferometrically reflected from the Fabry Perot cavity is in use modulated by the signal from the acoustic field incident on the acoustically sensitive surface at that location. In an aspect, there is provided a method of generating a fitted interferometer transfer function $ITF_F$(x,y) for each of a plurality of measured interferometer transfer functions ITFc(x,y), optionally obtained in accordance with the preceding aspect, by fitting a template interferometer transfer function $ITF_T$ to each ITFc(x,y).

According to another non-claimed aspect, there is provided a computer readable medium comprising instructions which when executed by one or more processors, cause the one or more processors to: implement the method for generating a tuning map of a sensor head as disclosed in above the above aspect, or to implement the method of generating fitted interferometer transfer functions for each of a plurality of measured interferometer transfer functions..

In order to effectively interrogate the sensor head in use to recover the modulation of the reflected beam power by the photoacoustic field incident on the sensing surface of the sensor head in use, the sensor head needs to be characterised by generating a tuning map of the sensor head. Generally, due to the manufacturing process of the Fabry Perot etalon, the thickness of the cavity, and therefore the optical path length of light in the cavity, can vary significantly across the sensing plane of the Fabry Perot etalon. In this way, the central wavelength of the ITF, and thus the determined bias wavelength for scanning and interrogating the sensor head, is shifted in wavelength space at addressable locations on the sensor head as the dimensions and response of the cavity varies. Thus, to operate the photoacoustic imaging apparatus, the ITF of the sensor head needs to be accurately characterised at addressable locations across the sensor head in a timely way. To ensure the sensed signal is accurate, this characterisation needs to be performed at regular intervals throughout the use of the apparatus.

The present inventors have realised that self-heating of the sensor head due to repeated exposure of the addressable locations to interrogation beams at different wavelengths to characterise the ITF at those locations causes the characterised ITF to become skewed and the results of the bias phase selection process to be inaccurate. Further, the optimum bias wavelength is typically selected to be substantially at an extrema (typically the maximum) of the derivative of an interferometer transfer function (ITF) for each addressable location , as at these wavelengths, the sensitivity and linearity of the sensor response is a maximum. However, the characterised ITF data can be inherently noisy due to different noise sources in the system, and can comprise a number of reflected power signal values at adjacent tuned wavelengths that can be significantly above and below the broader trend of the sensor head ITF, which can lead to significant errors in the selection of the bias wavelength at locations across the sensor head. Errors in bias wavelength selection lead to very poor quality, possibly unusable, data for image recovery. Repeated ITF characterisation could mitigate non-systemic random error sources (but this will have a limited effect as many errors in the process are systemic). However, accurate characterisation of the ITF for each addressable location requires the tuning of the interrogation laser to all wavelengths across the free spectral range of the etalon for all addressable locations. As a result, the time taken to characterise and calibrate the sensor head can be extremely long, making its use impractical.

To characterise the sensor head on a practicable timescale and in such a way as to facilitate selection of an accurate bias wavelength for addressable locations of the sensor head (and thus recovery of a good quality image in use) the above-described aspect of the present disclosure provides for the use of a template ITF for the sensor head to be fitted to a generated ITF characterised for addressable locations of the sensor head. The template ITF represents an idealised, and noise-free shape of a response of the Fabry Perot etalon for all locations across the sensor head, and may be generated empirically based on characterisation data, semi-empirically, or theoretically for the etalon design. By fitting the template ITF to a generated ITF in wavelength and reflected power space, for example, by translating the template ITF to aligning with the peak minimum of the generated ITF (or by using a suitable minimisation method), a noise free characterisation of the response of the etalon can be achieved in a series of ITFs fitted for the response of each addressable location. Further, surprisingly, this approach enables accurate characterisation results to be obtained quickly, and excellent images to be obtained in use, by generating only a coarse ITF for addressable locations of the sensor head for a range of different phase bias control methods. For example, where phase bias control is achieved by wavelength control, by tuning the interrogation laser to only a subset, even a small subset (rather than a large subset or all of the tuneable wavelengths of the ITF across its free spectral range) the characterisation of the sensor head can be performed very quickly with minimal scanning and tuning. By tuning the laser in steps, each separated by the relatively long time required to complete a 2D scan, the laser tuning initialisation and settling times form a much smaller proportion of the total time required to acquire the ITF map. This in turn avoids significant self-heating at each point, thus avoiding distortion of the generated ITF. The fitting of the template ITF to a relative coarse but accurate generated ITF provides for very accurate fitted ITFs that better reflect the true response of the sensor head, such that the bias wavelength selection and the resulting tuning map are very accurate.

Optionally, the phase control means may be configured to control the phase difference of the light of the one or more interrogation beams in the cavity by one or more of:

• tuning a wavelength of light produced by the wavelength tunable source;

• controlling a thickness of the cavity;

• controlling a refractive index of the cavity.

Optionally, the controller is further configured to store in the computer readable medium the respective bias phase, $\varphi_b(x,y)$, for the apparatus at each addressable location (x,y) as scanning wavelength data, or as scanning thickness data, or as scanning refractive index data. In this way, the tuning map may be generated and stored and used to control the phase difference of light in the cavity by one or more appropriate phase control mechanisms implemented by the phase control means.

Optionally, the controller is configured to control the beam directing means to direct the one or more interrogation beams to all of the addressable locations (x,y) of the sensor head and to generate an interferometer transfer function ITF$_G$ (x,y) for all of the addressable locations (x,y).

Optionally, only a coarse interferometer transfer function ITF$_G$ (x,y) is generated for the addressable locations by the characterisation phases ($\varphi_{1...n}$) being selected at spaced, relatively coarse, intervals within the range of phases between two minima of the ITF, when compared with the tuning steps to which the phase can be tuned within the range.

Optionally, to generate a template interferometer transfer function ITF$_T$, the controller is further configured to: select a subset of addressable locations based upon a quality metric of their corresponding ITFcs; average the ITF$_G$s corresponding to the subset of addressable locations to generate the ITF$_T$. In this way, the template ITF can be generated empirically from an average of a selected subset of the best quality generated ITFcs.

Optionally, to perform the fitting process in a highly efficient manner, a template ITF function (ITF$_T$) is formed from a subset of the generated ITF$_G$s. This template ITF$_T$ is then rescaled to fit the generated ITF$_G$s at each spatial point to generate fitted ITF$_F$s for addressable locations of the sensor head. This approach is robust, accurate and computationally very efficient, the latter because it avoids the use of a non-linear fit.

Optionally, to average the ITF$_G$s corresponding to the subset of addressable locations to generate the ITF$_T$ the controller is further configured to: calculate the mean of the ITF$_G$s. Optionally, a model is fitted to the mean ITF$_G$. Optionally, the model is a Lorentzian Airy function. In this way, a semi-empirical, or even a theoretical approach may be taken.

In some circumstances, the mean ITF$_G$ may not not a sufficiently accurate representation of the true ITF. In this case a model (eg the Lorentzian Airy function) may be fitted (using a nonlinear fit) to the mean ITF$_G$.

Optionally, the quality metric of each ITF$_G$ is a visibility metric, the visibility metric defined as (P$_{rmax}$-P$_{rmin}$)/(P$_{rmax}$+P$_{rmin}$), wherein P$_{rmax}$ is the maxima of the ITF$_G$ and P$_{rmin}$ is the minima of the ITF$_G$ This approach can be used as the metric for determining the subset because it is robust to noise and can be computed efficiently.

Optionally, to fit a template interferometer transfer function ITF$_T$ to each ITF$_G$ (x,y) to generate a fitted interferometer transfer function ITF$_F$(x,y) for each of the plurality of addressable locations (x,y), the controller is further configured to: align the minima of the ITF$_T$ with the minima of each ITF$_G$ (x,y), apply a vertical offset and multiplier such that ITF$_T$ is fitted to each ITF$_G$ (x,y). Since this is a simple 1-axis scaling operation and avoids using a nonlinear fit, it is efficient and fast. This fitting process yields in a map of noise-free ITFs, one for each x,y position on the sensor.

Optionally, the sensor head is formed as a planar Fabry Perot etalon, wherein the etalon comprises the addressable locations (x,y) of the sensor head onto which the interrogation beam can be directed in use.

Optionally, the sensor head is formed to have an array of Fabry Perot interferometric cavities with plano-convex geometry, wherein individual cavities correspond to the addressable locations (x,y) of the sensor head onto which the interrogation beam can be directed in use.

The plano-convex cavity (Figure 3B) is a more recent development and has the potential to offer significantly higher sensitivity since it avoids beam walk-off thereby allowing many more round trips within the cavity thus increasing its Q-factor. By providing a sensor head having addressable locations corresponding to plano-convex cavities (as shown in Figure 3B) offers significantly higher sensitivity and signal response since it avoids beam walk-off (i.e. the beam is contained at the addressable location), thereby allowing many more round trips within the cavity thus increasing its Q-factor.

Optionally, the wavelength tuneable source is arranged to generate a plurality of beams. Optionally, the plurality of beams are arranged in a line. Optionally, a subset of the plurality of beams corresponds to a subset of the plurality of addressable locations. The use of multiple beams, from a single tuneable wavelength source or even from multiple tuneable wavelength sources, further speeds up the scanning and interrogation process. Plural beam directing means may be provided to direct each of the multiple beams onto different addressable locations of the sensor head. The beam directing means may be arranged to scan the multiple beams in a line, allowing faster interrogation of grouped regions of the sensor head.

As only a relatively coarse ITF is generated for locations of the sensor head by tuning the interrogation wavelength source to a selected subset of tuneable wavelengths across the free spectral range of the etalon, much less of the time taken to characterise the sensor head is taken up by the relatively slow tuning and settling times of the laser. Conversely, the scanning of the sensor head now forms a much greater proportion of the total pre-tuning time. Optionally, when using a multi-beam system, the fitting of the IFT$_T$ to the generated ITF$_G$s may be performed for a subset or only one of the beams within a small region of the sensor head. Where the beams are arranged and swept in a line, only one or a subset of the beams may generate an ITF$_G$ for fitting. In doing so, a coarse pre-tuning map is generated and the computation time required to achieve this can be reduced by N times (wherein N is the number of beams). In addition, the computation time required to perform the grouping process and form the scan-plan is also reduced by N.

Implementing the above approaches has enabled a significant reduction of the time required to complete the entire pre-tuning process. Using a single or multi-beam scanner, the tuning map characterisation of the bias phases for the addressable locations of the sensor head has been reduced to at most 1 minute, and even down to 6 seconds for a 16 beam scanner.

Optionally, the controller is further configured to: use the determined the bias wavelengths to predict bias wavelengths associated with the addressable locations separate to the subset of addressable locations.

Optionally, the controller is further configured to generate a look-up table grouping the addressable locations with determined and/or predicted bias wavelengths. Optionally, the controller is further configured to generate a scan-plan, the scan-plan comprising an ordered list of the plurality of addressable locations, wherein each addressable location with the same determined and/or predicted bias wavelengths is grouped together. Optionally, the controller is further configured to select the determined bias wavelength, $\lambda_b$, for the addressable locations (x,y) to be substantially at an extrema of the derivative of the fitted interferometer transfer function $ITF_F$ for each addressable location (x,y).

Thus the scanning phase data can be stored in any appropriate way that allows region-by-region, wavelength-by-wavelength scanning in use. It can be stored as a simple tuning map for all addressable locations, and post-processed in the controller to group them into regions of common phase and generate a scan plan to control the beam directing means and the phase control means to carry out the tuning and scanning across the addressable locations region-by-region and phase-by-phase. Alternatively, the tuning map data may be pre-processed such that the scanning phase data may be stored as a grouped regions of common wavelength (e.g. in a look up table) for post processing into a scan plan, or as a scan plan itself.

Optionally, to adjust the determined bias wavelength, $\lambda_b$, to compensate for local or bulk changes in the optical path length in the cavity in use, such as due to variations in sensor head temperature and applied pressure since the tuning process, the controller is further configured to: in use, monitor the reflected power of the one or more reflected beams at one or more of the addressable locations (x,y) for bias tracking; for each of the one or more addressable locations (x,y) for bias tracking: compare the reflected power with a reflected power expected for the current bias wavelength $\lambda_b$ from the ITF generated during the tuning process for apparatus; and determine an adjustment $\delta\lambda_b$ to the current bias wavelength $\lambda_b$ needed to compensate for the change in the reflected power from the cavity; and, update the stored values of the determined bias wavelength, $\lambda_b$, in the scanning wavelength data for addressable locations (x,y) of the sensor head to a compensate bias wavelength value based on the determined adjustment $\delta\lambda_b$ to the currently stored bias wavelength $\lambda_b$ for the one or more addressable locations (x,y) for bias tracking.

In this way it is possible to track changes in the bias wavelength and maintain the sensor at optimum sensitivity in the presence of self-heating or external temperature and pressure changes.

According to another non-claimed aspect, there is provided methods and apparatuses for use in ultrasound optical sensing with respect to a sample in which an acoustic field is generated in response to a stimulus, for tracking and compensating for changes in phase bias. The apparatus comprises: a sensor head having an acoustically sensitive surface arranged as a reflective surface of a Fabry Perot interferometric cavity, wherein, when the acoustically sensitive surface is placed against the sample, in use, the acoustic field generated in response to said stimulus is incident upon said acoustically sensitive surface to modulate the optical path length in the cavity with a signal indicative of the acoustic field; a light source, optionally wavelength tuneable, for generating one or more interrogation beams of electromagnetic radiation; beam directing means to direct the one or more interrogation beams onto said acoustically sensitive surface; phase control means for controlling the phase difference of the light of the one or more interrogation beams in the cavity; detection means configured to receive and determine one or more values representative of the power of the reflected one or more interrogation beams; and computer readable medium storing phase data representative of a phase value for controlling the phase control means, the phase data representing a determined bias phase, $\varphi_b$, for the sensor head, selected to be a phase difference of the light field in the cavity at which the power of the interrogation beam interferometrically reflected from the Fabry Perot cavity is in use modulated by the signal from the acoustic field incident on the acoustically sensitive surface at that location. The method is implemented in a controller to adjust the determined bias phase, $\varphi_b$, to compensate for local or bulk changes in the optical path length in the cavity in use, such as due to variations in sensor head temperature and applied pressure since the tuning process, the controller being configured to: in use, monitor the reflected power of the one or more reflected beams; and determine, from the reflected power, an adjustment $\delta\varphi_b$ to the current bias phase $\varphi_b$ needed to compensate for the change in the reflected power from the cavity; and, update the stored values of the determined bias phase, $\varphi_b$, in the phase data to a compensated bias phase value based on the determined adjustment $\delta\varphi_b$ to the currently stored bias phase $\varphi_b$. Determining, from the reflected power, may comprise comparing the reflected power with an expected reflected power expected for the current bias phase $\varphi_b$ from an ITF generated from a tuning process for the apparatus.

According to another non-claimed aspect, there is provided a computer readable medium comprising instructions which when executed by one or more processors, cause the one or more processors to implement the method for performing ultrasound optical sensing as disclosed in above the above aspect.

In this way, it is possible to track changes in the bias wavelength and maintain the sensor at optimum sensitivity in the presence of self-heating or external temperature and pressure changes. As is apparent from the present disclosure, the bias tracking method and apparatus disclosed herein can be implemented independent of any need to scan a sensor head (or focal position) for imaging a target. For example, the phase bias tracking method disclosed herein is applicable in a non-imaging ultrasound optical sensor such as a single fibre sensor with a Fabry Perot cavity bonded to the end. Alternatively, the phase bias tracking method disclosed herein is applicable to the imaging ultrasound optical sensor in which the interrogation beam is scanned across addressable locations of the sensor head, and reflected signal power is detected to recover an image from the modulated signal indicative of the acoustic field.

Thus, the beam directing means may be configured to direct the one or more interrogation beams onto addressable locations (x,y) across said acoustically sensitive surface; the detection means is configured to receive and determine one or more values representative of the power of the reflected one or more interrogation beams from the addressable locations (x,y); and the phase data is stored as scanning phase data representative of a set of phase values for controlling the phase control means for addressable locations (x,y) of the sensor head, the scanning phase data relating: addressable locations (x,y) of the sensor head onto which the one or more interrogation beams can be directed in use; and a respective determined bias phase, $\varphi_b$, for the sensor head at given addressable locations (x,y).

The controller may be configured to monitor the reflected power at a single addressable location (x,y) for bias tracking, and to update the stored values of the determined bias phase, $\varphi_b$, for all addressable locations (x,y) of the sensor head based on a determined adjustment $\delta\varphi_b$ to the current bias phase $\varphi_b$ for the single addressable location (x,y) for bias tracking. In this way, single point bias tracking can be used to compensate the phase bias for changes in the cavity thickness that affect all addressable locations across the sensor head, such as global temperature changes or pressure changes. This single point bias tracking can also be used where only a single point measurement is being made to monitor the acoustic field incident at a single addressable location over time.

The controller may be configured to monitor the reflected power at a plurality of addressable locations (x,y) for bias tracking, and to update the stored values of the determined bias phase, $\varphi_b$, for all addressable locations (x,y) of the sensor head based on the determined adjustments $\delta\varphi_b$ to the current bias phase $\varphi_b$ for each of the plurality of addressable location (x,y) for bias tracking. To update the stored values of the determined bias phase, $\varphi_b$, for all addressable locations (x,y) of the sensor head based on the determined adjustments $\delta\varphi_b$ to the current bias phase $\varphi_b$ for each of the plurality of addressable location (x,y) for bias tracking, the controller may be configured to: upsample and interpolate the determined adjustments $\delta\varphi_b$ to the current bias phase $\varphi_b$ for each of the plurality of addressable location (x,y) for bias tracking, to obtain a determined adjustment $\delta\varphi_b$ to the current bias phase $\varphi_b$ for all of the addressable locations (x,y) across the sensor head. In this way, multi-point bias tracking can be used to compensate the phase bias for changes in the cavity thickness that vary spatially and in their effect on the phase bias at different addressable locations across the sensor head, such as local temperature changes or pressure changes.

Viewed from another aspect, the present invention provides methods and apparatuses for use in ultrasound optical imaging with respect to a sample in which an acoustic field is generated in response to a stimulus. The apparatus comprises: a sensor head having an acoustically sensitive surface arranged as a reflective surface of a Fabry Perot interferometric cavity, wherein, when the acoustically sensitive surface is placed against the sample, in use, the acoustic field generated in response to said stimulus is incident upon said acoustically sensitive surface to modulate the optical path length in the cavity with a signal indicative of the acoustic field; a light source, optionally wavelength tuneable, for generating one or more interrogation beams of electromagnetic radiation; beam directing means to direct the one or more interrogation beams onto said acoustically sensitive surface; phase control means for controlling the phase difference of the light of the one or more interrogation beams in the cavity by controlling one or more of: a thickness of the cavity; and a refractive index of the cavity; detection means configured to receive and determine one or more values representative of the power of the reflected one or more interrogation beams; and computer readable medium storing phase data representative of a set of phase difference values for controlling the phase control means, the phase data representing a determined bias phase, $\varphi_b$, for the sensor head at given addressable locations (x,y), selected to be a phase difference of the light field in the cavity at which the power of the interrogation beam interferometrically reflected from the Fabry Perot cavity is in use modulated by the signal from the acoustic field incident on the acoustically sensitive surface at that location. The method is implemented by a controller configured to: access the phase data stored in the computer readable medium to obtain a phase bias, $\varphi_b$, of the sensor head; control the phase control means to control one or more of the thickness and refractive index of the cavity at based on the phase bias, $\varphi_b$, to control the phase difference of the light of the interrogation beam in the cavity; interrogate, using the beam directing means, the sensor head; and receive, from detection means, a value representative of the power of the reflected interrogation beam; and form a signal indicative of the signal modulated on the reflected one or more interrogation beams by the acoustic field incident on the acoustically sensitive surface.

Viewed from another aspect, the present disclosure provides a computer readable medium comprising instructions which when executed by one or more processors, cause the one or more processors to implement the method for performing ultrasound optical imaging as disclosed in above the above aspect.

In this way, the phase difference of light can be controlled in the cavity by biasing the thickness and/or refractive index of the cavity. This may provide advantages independently or alongside other phase control mechanisms, such as wavelength tuning. Indeed, phase control of light in the cavity may be achieved by biasing one or more of the thickness of the cavity, the refractive index of the cavity, and wavelength of the interrogation beam. These phase control variables may be controlled independently of each other or in combination.

As will be appreciated, the phase control method and apparatus disclosed herein can be implemented independent of any need to scan a sensor head for imaging a target. For example, the phase control method disclosed herein is applicable in a non-imaging ultrasound optical sensor such as a single fibre sensor with a Fabry Perot cavity bonded to the end. Alternatively, the phase control method disclosed herein is applicable to the imaging ultrasound optical sensor in which the interrogation beam is scanned across addressable locations of the sensor head, and reflected signal power is detected to recover an image from the modulated signal indicative of the acoustic field.

The phase control means may be configured to control the interrogation light source to generate a heating pulse of controlled energy to heat the sensor head structure and locally heat the cavity to expand the cavity thickness to produce a phase difference of light in the cavity corresponding to the bias phase before interrogating the addressable location to measure the reflected power.

The cavity of the sensor head may be configured such that it expands and contracts responsive to operation of the phase control means, and wherein the phase control means is configured to control the cavity to expand or contract the cavity thickness to produce a phase difference of light in the cavity corresponding to the bias phase before interrogating the addressable location to measure the reflected power.

The scanning phase data may be stored as thickness data representing a determined bias thickness, $t_b$, for the cavity; or, wherein the phase data is stored as refractive index data, representing a respective determined bias refractive index, $n_b$, for the cavity. Alternatively, where more than one phase control variable is used to control the phase difference of light in the cavity, the scanning phase data may be stored as a combination of these variables, such as optical thickness data representing a combination of a bias thickness and a bias refractive index needed to obtain a desired phase bias.

BRIEF DESCRIPTION OF THE DRAWINGS

Certain preferred embodiments of aspects of the invention will now be described, by way of example only, and with reference to the accompanying drawings, in which:

Fig. 1 is a perspective view of a planar Fabry Perot etalon provided in a sensor head;

Fig. 2 is a schematic illustration of an apparatus for performing photoacoustic tomography;

Fig. 3A is a sectional view of a planar Fabry Perot etalon;

Fig. 3B is a sectional view of a Fabry Perot interferometric cavity with plano-convex geometry;

Fig. 4A is an illustrative graph of an ITF in phase difference space showing the relationship between reflected interrogation beam power and the phase difference between the optical fields reflected from the two mirrors of the Fabry Perot interferometer for an interrogation beam for a Fabry Perot sensor, including an indication of a selection of a bias point;

Fig. 4B is a plot of an ITF in wavelength space showing the relationship between reflected interrogation beam power and the wavelength of an interrogation beam for a Fabry Perot sensor, including an indication of a selection of a bias wavelength;

Fig. 5 is a process flow diagram illustrating step for generating a tuning map and scan plan for use when performing photoacoustic tomography;

Fig. 6 illustrates an example method for performing ultrasound optical imaging with respect to a sample;

Fig. 7 illustrates an example method for generating a tuning map of a sensor head, the sensor head for use in performing ultrasound optical imaging with respect to a sample;

Fig. 8 illustrates an example method for use in performing ultrasound optical imaging with respect to a sample for tracking and compensating for changes in phase bias; and

Fig. 9 illustrates an example method for use in performing ultrasound optical imaging with respect to a sample for controlling a thickness of a cavity of a Fabry Perot interferometer sensor to control the phase difference of the light of the interrogation beam in the cavity.

DESCRIPTION OF THE EMBODIMENTS

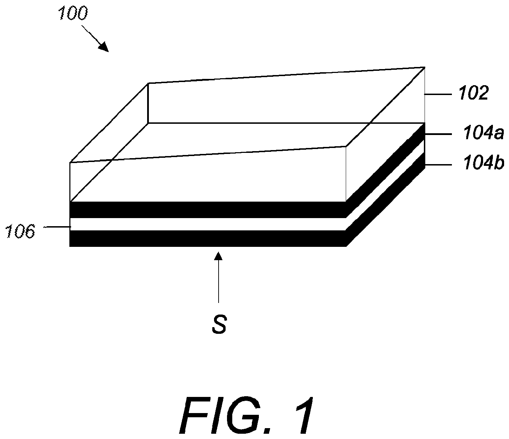

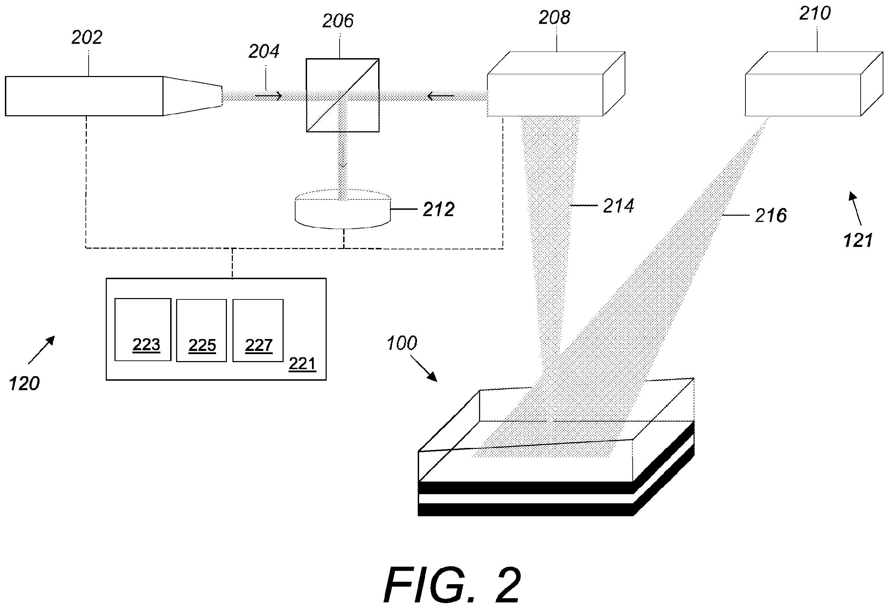

A sensor head and a ultrasound optical imaging apparatus incorporating the sensor head will now be described with reference to Figures 1 and 2. While the ultrasound optical imaging apparatus described herein are applied for photoacoustic tomography medical imaging of tissue samples in particular, it is to be understood that this is presented by way of example only, and that the ultrasound optical imaging apparatuses and methods disclosed herein are applicable to sensing acoustic fields generated by other stimuli, and in different, non-tissue samples, for other applications.

Apparatus 200 for performing photoacoustic tomography with respect to the sample (not shown) that receives a pulse of excitation electromagnetic radiation and generates an acoustic field in response to said pulse, comprises generally, a sensor head 100, an excitation light source 121 and an interrogation assembly 120.

The excitation light source 121 is arranged to provide through the sensor head 100 an excitation light beam 216 to be absorbed by a tissue sample and to generate a photoacoustic signal field in the tissue to be detected using the sensor head 100 and interrogation assembly 120. The stimulus of the acoustic (ultrasound) field for readout by the sensor head 100 and interrogation assembly 120 can be any suitable stimulus, such as a piezo transducer or a MEMS device, and is not limited to being generated by a pulsed laser using the photoacoustic effect. Further, described in relation to sensing an acoustic field in a tissue sample, this also is not limiting, and the optical ultrasound readout technique, implemented by the sensor head 100 and interrogation assembly 120, may be applied to sense acoustic fields in a range of media and for a range of different applications, such as for non-destructive testing.

The excitation light source 121 may provide a pulsed light output. The excitation light source 121 may be provided by at least an Optical Parametric Oscillator (OPO) driven by an appropriate source of coherent light, such as an Nd:YAG pulsed laser. In this way, the OPO can be used to change the excitation wavelength of the excitation light source 121 to different wavelengths that are selectively absorbed by the tissue structure, such that different structural and functional features of the tissue can be studied using the apparatus 200.

Figure 1 illustrates in more detail the a planar Fabry Perot etalon for use in the sensor head 100, the Fabry Perot etalon may represent the sensor head 100 or at least the sensing element thereof. The sensor head 100 comprises a wedged transparent polymer backing stub 102 on to which a multilayer sensing structure of the Fabry Perot etalon is vacuum deposited, such as by chemical vapour deposition (CVD). This includes a spacer 106, typically formed of Parylene polymer film 10-50 $\mu$m thick, depending upon the acoustic bandwidth required, sandwiched between two highly reflective mirrors 104a, 104b typically provided by dichroic dielectric thin film mirrors. The spacer 106 provides the optical cavity of the Fabry Perot interferometer over which multiple reflections of light travel and from which escaping reflected light can constructively or destructively interfere depending on the phase difference between the optical fields reflected from the two mirrors of the Fabry Perot interferometer for the light in the cavity. While as shown in Figure 1, the resonant Fabry Perot cavity is planar in geometry, as will be seen below, other cavity structures are suitable if they can provide a resonant effect on light in the cavity. Such alternative structures include plano-convex microresonator arrays, inverted plano-convex microresonator arrays, for example, having mirrored surfaces.

The mirrors 104a, 104b, are designed to be highly reflective (i.e. reflects at least 95% of power) in a first wavelength range thus forming with the spacer 104 a high finesse Fabry Perot cavity in this wavelength range but highly transmissive in a second wavelength range. Preferably the first wavelength range is between 1500-1700nm and the second wavelength range is 600nm-1200nm.

In use, the sensor head 100 is placed such that a sensing surface thereof S is faced against and acoustically coupled to the tissue sample to be imaged (not shown). A coupling gel may be used to keep ensure the acoustic field is conveyed from the tissue to the surface S.

The second wavelength range enables the excitation laser pulses from the excitation light source 121 in the near infrared (NIR) window, where biological tissues are relatively transparent, to be transmitted through the sensor head 100 into the tissue. The photoacoustic signals generated by the absorption of the laser energy propagate back to the surface S where they modulate the optical thickness of the spacer 106 and thus the reflectivity of the Fabry Perot sensing structure in the 1500-1700nm wavelength.

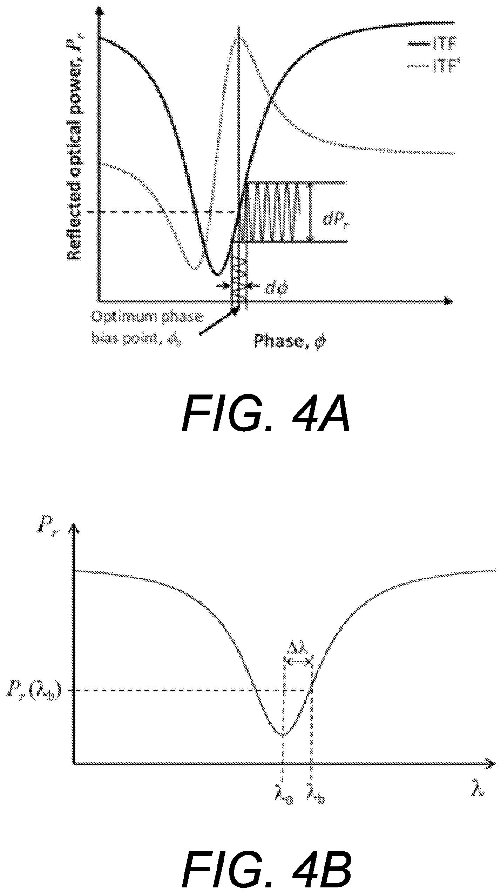

The Fabry Perot etalon of the sensor head 100, having a high finesse, is selectively reflective having a narrowband reflection spectrum, as characterised by the Interferometer Transfer Function ITF of locations of the sensor head, which relates the power of the light reflected by the Fabry Perot etalon across different wavelengths in the free spectral range of the cavity, at a given location on the etalon. Figure 4 illustrates the ITF of an example Fabry Perot etalon of the sensor head 100 in phase difference space (see Figure 4a) and in wavelength space (see Figure 4b).

The central phase point $\varphi_0$ or central wavelength $\lambda_0$ of the Interferometer Transfer Function at locations across the sensor head is dependent at least in part on the optical path length of light in the cavity, as defined by the thickness of the spacer 106 at those locations. The acoustic signals from the tissue incident on the sensing surface S of the sensor head 100 modulate the optical path length of the cavity of the Fabry Perot etalon at different locations across its surface by acoustic resonance (i.e. stimulation) of at least the thickness of the spacer 106. This in turn modulates the central phase point $\varphi_0$ and central wavelength $\lambda_0$ of the etalon ITF in dependence on the acoustic field incident at that location.

Thus, for a single wavelength of light of the ITF, particularly where the ITF slope is high, the reflected power of light of that wavelength is also modulated as the ITF is moved back and forth in wavelength space by the acoustic modulation of the spacer 106. In order to achieve a consistent and desired maximum sensitivity of the reflected power $P_r$ to modulation by $dP_r$ by the acoustic field, the phase difference of light in the cavity is biased in use accordingly by a bias phase $\varphi_b$. This may be selected to be the phase difference between the optical fields reflected from the two mirrors of the Fabry Perot interferometer at which the magnitude of $dP_r$ is maximised and at which the response is most linear (typically where the value $\varphi$ where the of the derivative of the ITF is at a maximum). As will be described below, the biasing of the phase can be achieved for example by operating the phase control means 222 to tune a wavelength tunable interrogation light source 202 to a bias wavelength $\lambda_b$ at which the phase difference of light in the cavity is at the bias phase $\varphi_b$. Alternatively, or in addition, the phase control means 222 may be configured to control the phase difference between reflections of the light of the interrogation beam in the cavity controlling a thickness of the cavity and/or by controlling a refractive index of the cavity.

The characterisation of the etalon ITF to calibrate the apparatus 200, and the determination and monitoring of the bias wavelength $\lambda_b$ for use in interrogating the sensor head, will be described in more detail later in the specification in relation to Figures 4, 5 and 7.

The interrogation assembly 120 is arranged to optically interrogate the sensor head at locations across the sensor head to reveal the modulation of the reflected power at that location, to enable an image of the photoacoustic field (specifically as it is affected by absorption within the structure of the sample being imaged) to be reconstructed using tomographic techniques.

The interrogation assembly 120 may comprise a tuneable coherent interrogation light source 202, beam directing means 208, a detection means 212 and a controller 221. The controller 221 is coupled to the interrogation light source 202, beam directing means 208, detection means 212 and phase control means 222 to provide control signals thereto, and to receive detection signals therefrom and is configured to control the interrogation assembly 120 to carry out the methods for performing photoacoustic tomography disclosed in the present application.

The interrogation light source 202 is arranged to provide a focussed beam of light 204 to beam directing means 208 which redirects the light to move a scanning beam 214 across the surface the Fabry Perot etalon of the sensor head 100. The interrogation light source 202 is configured to be tuneable by the phase control means 222 responsive to control by the controller 221 to tune the wavelength of the light 204 to a bias wavelength $\lambda_b$ at which a bias phase $\varphi_b$ of light in the cavity is obtained. The Fabry Perot etalon of the sensor head 100 is designed and the interrogation light source 202 may be selected such that the interrogation light source 102 is tuneable across the free spectral range of the Fabry Perot etalon. While as discussed above, the phase difference of the light in the cavity is tuned to a bias phase by the phase control means 222 tuning the wavelength of light of the interrogation light source 202, the phase difference of light in the cavity may be controlled to achieve a desired phase bias by the phase control means 222 adjusting the phase difference by other means, such as by adjusting one or more of the wavelength of light, the optical path length of light in the cavity and the refractive index of the cavity, by appropriate means.

The interrogation light source 202 may be a tuneable laser emitting coherent light in a range around 1550nm. The interrogation light source 202 is coupled to controller 221 and is configured to be tuned thereby to transmit light at a wavelength based on a control signal sent from the controller 221.

The beam directing means 208 is a controllable beam directing means operable to direct the one or more interrogation beams onto addressable locations (x,y) across said acoustically sensitive surface S in response to control by the controller 221. The beam directing means 208 is configured to redirect the focused beam 204 onto the Fabry Perot etalon of the sensor head 100 to point the scanning beam 214 to be incident on the Fabry Perot etalon at any one of a plurality of addressable locations across the plane of the Fabry Perot etalon, which may be specified or represented as values x and y of the x and y axes of a set of planar coordinates on the etalon. The beam directing means 208 is configured to steer the scanning beam 214 to a given addressable location (x,y) based on a control input received from controller 220. The addressable locations (x,y) of the interrogation assembly 120 may be specified by stepwise increments of the redirection angles in x and y achievable by the beam directing means, and/or by increments or control values specified by the controller 220. The redirection of the focussed beam 204 may be achieved in the beam directing means by one or more steering mirrors, lenses, microlenses or other appropriate optical components attached to one or more micromechanical control devices, servos, MEMS controllers or galvanometers. Instead of redirecting one or more beams in free space onto and across the Fabry Perot interferometer, the beam directing means may be an optical fibre or an optical fibre bundle and the sensor head/Fabry Perot interferometer may be mounted on an optical fibre or fibre bundle. In this way, where an optical fibre bundle is used, the location of the different fibres may define the addressable locations. The beam directing means 208 may thus be usable to interrogate addressable locations of the interferometer without needing to be actively controlled to direct the beam accordingly.

The detection means 212 is arranged to sense the power of the light reflected from the sensor head and to provide a signal indicating the measured reflected power to the controller 221. The interrogation assembly 120 may comprise a beam splitter 206, and/or one or other appropriate optical components which may be arranged to focus the light beam onto the sensor head 100 and to redirect light reflected from the sensor head 100 onto the detection means 212. The detection means 212 may be any component configured to absorb incident light in the tuneable range of the interrogation light source and provide a signal output indicative of the power of the incident light. The detection means 212 may be a photodetector, an active pixel sensor, a charged coupled device, or a bolometer, for example.

The controller 221 may comprise a computer readable medium, which may be a random access memory (RAM) such as one or more volatile or non-volatile memory solid state memory units, such as flash memory. The controller 221 may also comprise one or more data processors.

The computer readable medium of the controller 221 stores scanning phase data 223. The scanning phase data 223 may be stored as a data array or a look up table or in any other suitable form.

The controller 221 also comprises an interrogation control module 225 and a sensor tuning module 227. The interrogation control module 225 and sensor tuning module 227 may be provided by one or more logical components implemented by one or more data processors of the controller 221 in use.

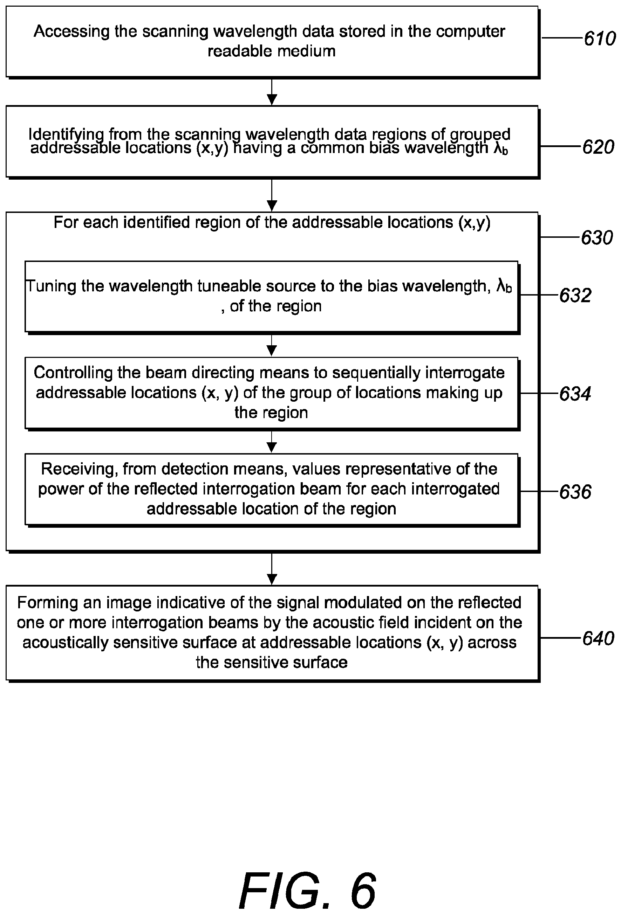

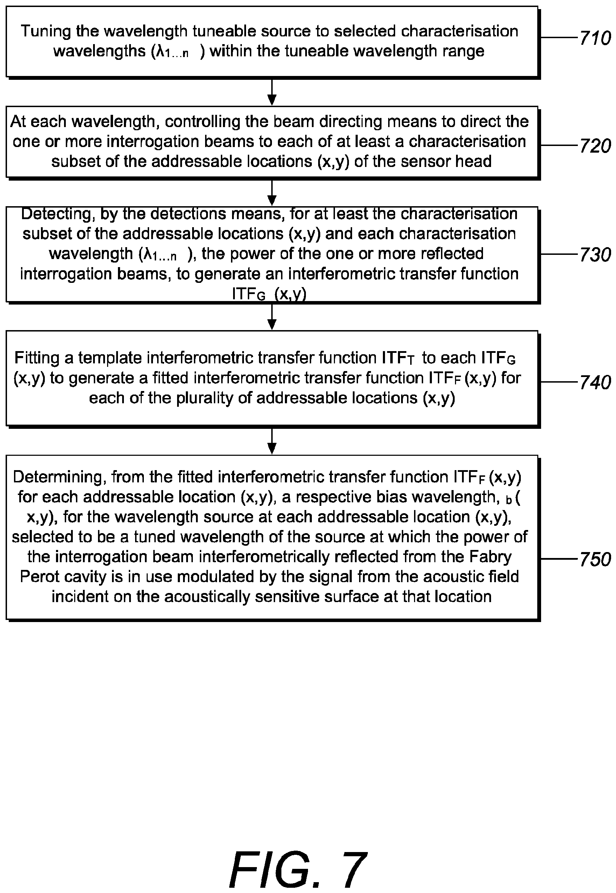

The controller 221 may comprise one or more data processors preconfigured to provide one or more of the said logical components. For example, one or more of the processors may be dedicated microcontroller or Field Programmable Gate Array-based components specifically designed to carry out certain steps of the methods described herein. The one or more data processors may comprise an FPGA, a microcontroller of a dedicated design, or any other appropriately configured data processing means of implementing the interrogation control module 225 and carrying out the method described below in relation to Figure 6. The one or more data processors may also comprise an FPGA, a microcontroller of a dedicated design, or any other appropriately configured data processing means of implementing the sensor tuning module 227 and carrying out the method described below in relation to Figure 7.

Alternatively, or in addition, the controller 221 may comprise one or more data processors configured only in use to provide one or more of the said logical components. For example, one or more of the processors may be a general-purpose processor (e.g. a Central Processing Unit) coupled to a memory comprising instructions for configuring the processor to carry out certain steps of the methods described herein. The computer readable medium of the controller 221 that stores the scanning wavelength data 223 may also store instructions which when executed by one or more data processors, cause one or more of the data processors to implement the interrogation control module 225 and carry out the methods described below in relation to Figure 6, 8 and 9. The computer readable medium of the controller 221 may also store instructions which when executed by one or more data processors, cause one or more of the data processors to implement the sensor tuning module 227 and carry out the method described below in relation to Figure 7.

The phase control means 222 controls the phase difference of light from the interrogation beam in the cavity by tuning a wavelength of light produced by the wavelength tunable source 202. That is, the phase control means 222 controls the tunable laser 202 to change the wavelength of light in the cavity, for example to a bias wavelength.

In this case, the scanning phase data 223 may be stored as scanning wavelength data, relating: addressable locations (x,y) of the sensor head; and a respective determined bias wavelength, $\lambda_b$, for the wavelength source at given addressable locations (x,y). The bias wavelength is selected to be a tuned wavelength of the source 202 at which the power of the interrogation beam interferometrically reflected from the Fabry Perot cavity 106 is in use modulated by the signal from the acoustic field incident on the acoustically sensitive surface S at that location.

Alternatively, or in addition, the phase control means 222 may control the phase difference of the light in the cavity by controlling a thickness of the cavity. This may be achieved by, for example, controlling the interrogation light source to generate a heating pulse of controlled energy to heat the sensor head structure and locally heat the cavity to expand the cavity thickness to a bias thickness before interrogating the structure to measure the reflected power. Alternatively, this may be achieved by the cavity may be made of a material that expands and contracts responsive to an external applied influence such as an electromagnetic field that can be applied by the phase control means to bias the cavity thickness. In this case the scanning phase data may be stored as scanning thickness data, relating: addressable locations (x,y) of the sensor head; and a respective determined bias thickness, $t_b$, for the cavity at given addressable locations (x,y). The bias thickness is selected to be a tuned thickness of the cavity at which the power of the interrogation beam interferometrically reflected from the Fabry Perot cavity is in use modulated by the signal from the acoustic field incident on the acoustically sensitive surface at that location.

Alternatively, or in addition, the phase control means may control the phase difference of the light in the cavity by controlling a refractive index of the cavity. This may be achieved by the cavity or sensor head being made of a material that changes the refractive index of the cavity responsive to an external applied influence such as an electromagnetic field that can be applied by the phase control means to bias the cavity thickness. In this case the scanning phase data may be stored as scanning refractive index data, relating: addressable locations (x,y) of the sensor head; and a respective determined bias refractive index, $n_b$, for the cavity at given addressable locations (x,y). The bias refractive index is selected to be a tuned refractive index of the cavity at which the power of the interrogation beam interferometrically reflected from the Fabry Perot cavity is in use modulated by the signal from the acoustic field incident on the acoustically sensitive surface at that location.

That is, the controller 221 and the phase control means 222 may control one or more of the wavelength, thickness and refractive index to control the phase difference of light in the cavity. While in the following description, the phase difference of light in the cavity is controlled by tuning the wavelength of the interrogation beam, it is to be understood that the principles can be equally applied by other means of phase control. Indeed, references in the following to 'scanning wavelength data' should be understood by the reader to be transposable to the broader phase-based tuning of the phase difference of light in the cavity.

As will be appreciated, the cavity thickness and refractive index phase control method and apparatus disclosed herein can be implemented independent of any need to scan a sensor head for imaging a target. For example, the phase control method disclosed herein is applicable in a non-imaging ultrasound optical sensor such as a single fibre sensor with a Fabry Perot cavity bonded to the end. Alternatively, as described herein in detail, the phase control method disclosed herein is applicable to the imaging ultrasound optical sensor in which the interrogation beam is scanned across addressable locations of the sensor head, and reflected signal power is detected to recover an image from the modulated signal indicative of the acoustic field.

As described in detail, the phase control is performed by controlling the wavelength of the interrogation laser. In this regard, the scanning wavelength data 223 is representative of a set of wavelength values of the wavelength tuneable source 202 for addressable locations (x,y) of the sensor head 100. Specifically, the scanning wavelength data 223 may relate:

- addressable locations (x,y) of the sensor head onto which the one or more interrogation beams can be directed in use; and

- a respective determined bias wavelength, $\lambda_b$, for the wavelength source at given addressable locations (x,y), selected to be a tuned wavelength of the source at which the power of the interrogation beam interferometrically reflected from the Fabry Perot cavity is in use modulated by the signal from the acoustic field incident on the acoustically sensitive surface at that location.

The scanning wavelength data 223 can be stored in any appropriate way for controlling the phase difference of the light in the cavity during readout.

The scanning wavelength data 223 can be stored in any appropriate way that allows region-by-region, wavelength-by-wavelength scanning in use.

For example, the data 223 can be stored in a database as tuples each relating a given addressable location (x,y) to the determined bias wavelength $\lambda_b$ for that addressable location, or as a bitmap or as a lookup table (LUT). It can be stored as a simple tuning map for all addressable locations, and post-processed in the controller to group them into regions of common wavelength and generate a scan plan to control the beam directing means and the tuneable laser to carry out the tuning and scanning across the addressable locations region-by-region and wavelength-by-wavelength. Alternatively, the tuning map data may be pre-processed such that the scanning wavelength data may be stored as a grouped regions of common wavelength (e.g. in a look up table) for post processing into a scan plan, or as a scan plan itself.

The scanning wavelength data 223 is used by the controller 221 to control the beam directing 208 means to direct the interrogation beam to a current addressable location (x,y) of the sensor head and to simultaneously control the phase biasing means 222 to tune the tuneable wavelength interrogation light source 202 to a bias wavelength $\lambda_b$ stored therein to achieve the desired bias phase $\varphi_b$ of light in the optical cavity for the current addressable location (x,y) of the sensor head 100. The controller may perform readout of the addressable locations (x,y) of the sensor head in an appropriate manner, such as by raster scanning. The scanning wavelength data 223 is simultaneously used by the controller 221 to coordinate the received power signal from the detection means 212 with the addressable location being interrogated.

As mentioned above, the scanning wavelength data 223 may be used by the controller 221 to tune the tuneable wavelength interrogation light source 202 and control the beam directing 208 means to perform interrogation of the addressable locations region-by-region and wavelength-by-wavelength for the different bias wavelengths, and to coordinate the received power signal from the detection means 212 with the addressable location being interrogated. It is to be understood however, that, in accordance with aspects of the invention, such region-by-region, wavelength-by-wavelength scanning may not be utilised, and instead another approach to scanning the sensor head to sense the reflected beam intensity may be used, such as raster scanning.

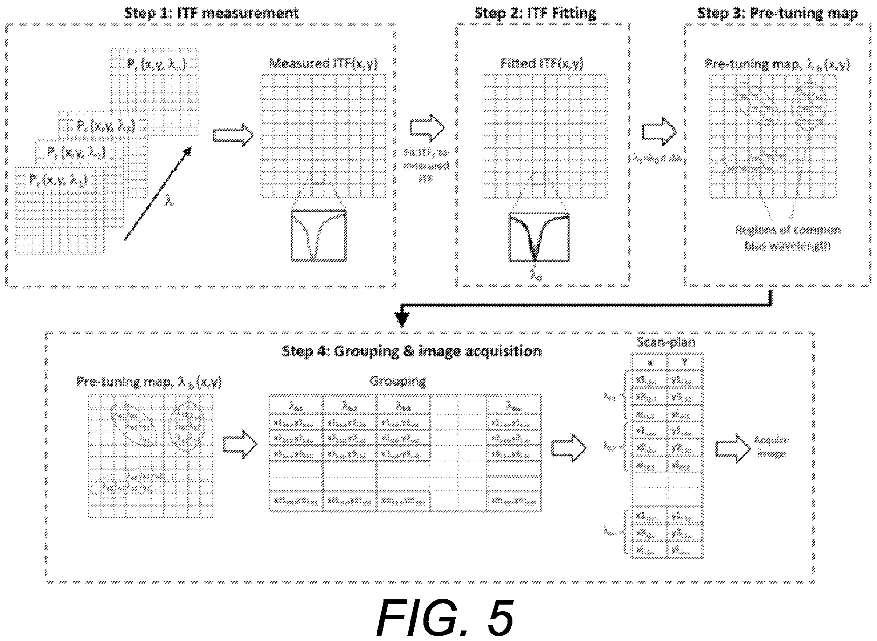

Methods of operating the above-described photoacoustic tomography apparatus to generate a tuning map of the sensor head to calibrate the apparatus, and to perform photoacoustic tomography, will now be described with reference to Figures 5, 6, 7, 8 and 9.

Referring to Figure 5, this broadly illustrates the beginning to end operational stages or steps of the photoacoustic tomography apparatus 200 to be performed in order calibrate the apparatus 200 and capture photoacoustic data to generate an image. There are four broad stages or steps as illustrated in Figure 5, which include:

- Step 1: ITF Measurement

- Step 2: ITF Fitting

- Step 3: Tuning map generation

- Step 4: Grouping and image acquisition

Broadly, steps 1, 2 and 3 above are performed by the apparatus 200 by operation of the sensor tuning module 227 to quickly provide high quality tuning map data for use in storing as scanning phase data 223, or, scanning wavelength data 223. The sensor tuning module 227 of the controller 221 is configured to operate the components of the interrogation assembly 120 to implement the methods for generating a tuning map disclosed in the present application. By this process, the sensor head 100 is characterised and the apparatus 200 is calibrated for operation, and this will be described below in relation to Figure 7.

Step 4 above is performed by the apparatus 200 by operation of the interrogation control module 225 to scan locations across the sensor head at a bias wavelength stored in the scanning wavelength data 223. In accordance with aspects of the invention, the scanning is performed region-by-region, wavelength-by-wavelength based on a scan plan derived from the scanning wavelength data 223.

The interrogation control module 225 of the controller 221 is configured to operate the components of the interrogation assembly 120 to implement the methods for performing photoacoustic tomography disclosed in the present application. By this process carried out by interrogation control module 225, the photoacoustic signals incident on the sensor head 100 are obtained for image generation, and this will firstly be described in relation to Figure 6.

With reference now to Figure 6, to implement the method for performing photoacoustic tomography disclosed in the present application, in use, the interrogation control module 225 of the controller 221 is configured to, in process activity 610, access the scanning wavelength data 223 stored in the computer readable medium.

Then, the interrogation control module 225 of the controller 221 is configured to, in process activity 620, identify from the scanning wavelength data 223 regions of grouped addressable locations (x,y) having a common bias wavelength, $\lambda_b$.

This may be achieved directly from the scanning wavelength data 223, if the scanning wavelength data 223 is stored as a region-by-region and wavelength-by-wavelength based scan plan containing complete instructions for the sequential controlling of the beam directing means 208 and the tuning of the tuneable light source 202. However, if the scanning wavelength data 223 is stored as a raw tuning map, e.g. of bias wavelength and addressable locations as a bitmap or as tuples, or as grouped regions of addressable locations (x,y) having a common bias wavelength, the interrogation control module 225 may be configured to process the scanning wavelength data 223 to generate a scan plan.

These regions of common $\lambda_b$ often form lines or curves that extend across a significant proportion of the entire sensor surface, and can sometimes resemble contours on a map.

The interrogation control module 225 of the controller 221 is further configured to, in process activity 630, for each identified region of the addressable locations (x,y), sequentially perform the process activities 632, 634 and 636 described below.

The interrogation control module 225 of the controller 221 is configured to, in process activity 632, tune the wavelength tuneable source to the bias wavelength, $\lambda_b$, of the region currently in process.

The interrogation control module 225 of the controller 221 is further configured to, in process activity 634, control the beam directing means to sequentially interrogate addressable locations (x, y) of the group of locations making up the region currently in process. Only the locations in the current group may be interrogated. More locations may be interrogated (it may be convenient to scan the beam in a raster or Lissajous pattern), but only values representative of the power of the reflected interrogation beam in for the current group of locations may be retained for subsequent storage and/or processing.

The interrogation control module 225 of the controller 221 is further configured to, in process activity 636, receive, from detection means, values representative of the power of the reflected interrogation beam for each interrogated addressable location of the region. The received values may be directly stored or post-processed and stored so that the photoacoustic signal modulating the reflected beam power at that addressable location can be retrieved and such that an image can be formed.

Optionally, the interrogation control module 225 of the controller 221 may be configured to, in process activity 640, form an image indicative of the signal modulated on the reflected one or more interrogation beams by the acoustic field incident on the acoustically sensitive surface at addressable locations (x, y) across the sensitive surface. As noted above, however, another component may carry out the formation of the image, or the scanning data may be stored and later processed to form the image. That is, the controller 221 may itself reconstruct an image derivable from the optically sensed photoacoustic field, for example using tomographic techniques, or the controller 221 may provide an output of the sensed power signal to a further image processing component (not shown) that is configured to form the image.

In accordance with the above-described method and apparatus, by scanning the focussed interrogation beam of laser light having a single wavelength (that is wavelength tuned to optimally set sensitivity at each scan point) on the ITF over the sensor head, its reflected power from the sensor head is modulated by the modulation of the optical path length of the cavity by the photoacoustic signal incident on the sensor head 100. In this way, the incident time dependent photoacoustic waves arriving at the sensor can be mapped in 2D as the interrogation assembly 121 and sensor head 100 can be regarded as a notional 2D ultrasound array, the aperture of which is defined by the dimensions of the scan region, the element size of the addressable locations (x,y), and the inter-element spacing by the laser spot size and the scan step size respectively. In order to reconstruct a 3D image from the detected time-resolved photoacoustic signals recorded over the surface of the sensor an efficient k-space backpropagation algorithm is used. Specifically, from the time-of-arrival of the signals, and with knowledge of the speed of sound, a 3D image of the tissue structure can then be reconstructed.

The approach described above in relation to Figure 6 minimises the number of times the laser wavelength (or other phase control means) has to be adjusted in order to complete a scan of the sensor head, and can therefore be completed on a timescale this is practical and usable in a clinical and life sciences setting. It is significantly more efficient than x-y raster scanning and adjusting the laser wavelength (or other phase control means) at each x-y position. However, it is important to scan any given set of spatial points of common $\lambda_b$ in the most efficient manner. This means minimising the distance between successive scan points. In other words it is important to start at an extremity of the area in which $\lambda_b$ is common (say, region 1) and scan it in small spatial steps (by determining the nearest neighbour at each point) avoiding large or random jumps. Once this area has been scanned, the interrogation beam is moved to another area of common $\lambda_b$, say region 2. The two regions are chosen such that they are in close proximity to avoid the need for a large step in moving from region 1 to region 2. The reason why minimising the step-size is important (both when scanning within a region and moving from one region to the next) is because the settling time for, e.g. the galvanometers, of the beam directing means 208 used to scan the laser beam scales with the step-size. For rapid acquisition, small steps are therefore required. The process of identifying the regions of common $\lambda_b$ and determining the optimum order of x,y positions (the "scan-plan") in order to scan most efficiently, both within a specific region of the same $\lambda_b$ and between regions of different $\lambda_b$ is called "grouping".

The process described above in relation to Figure 6 represents the implementation by the apparatus 200 of step 4 as shown in Figure 5, by which the scanning wavelength data 223 is grouped into regions and the sensor head 100 is scanned for image generation.

Now, with reference to Figure 7, an implementation by the apparatus 200 of steps 1, 2 and 3 of Figure 5 to quickly provide high quality tuning map data for use in storing as scanning wavelength data 223 will be described. The process shown in Figure 7 is performed by the apparatus 200 by operation of the sensor tuning module 227, when the sensor head 100 is not being actuated by a photoacoustic field (i.e. the optical thickness of the cavity and the reflected optical power are not modulated) or otherwise being deformed by applied pressure or heated by an external heat source that would change the thickness of the optical cavity.

The sensor tuning module 227 of the controller 221 is configured to operate the components of the interrogation assembly 120 to implement the methods for generating a tuning map disclosed in the present application. By this process, the scanning wavelength data 223 is generated by determining the bias wavelength for each addressable location such that the sensor head 100 is characterised and the apparatus 200 is calibrated for operation.

Before a reflected photoacoustic signal at a point on the surface of the Fabry Perot etalon can be measured, the bias phase at that point should be identified. The bias phase, $\varphi_b(x,y)$ is selected to be a tuned phase difference of light of the interrogation beam in the cavity at which the power of the interrogation beam interferometrically reflected from the Fabry Perot cavity is in use modulated by the signal from the acoustic field incident on the acoustically sensitive surface at that location. The bias phase difference is represented by the bias wavelength $\lambda_b(x,y)$ for the tunable wavelength source.

Since the thickness of the cavity varies from point to point due to manufacturing variations, this necessitates determining $\varphi_b$ at each point to generate a tuning map of the surface.

The optimum bias phase is the phase difference of light in the cavity at which the sensitivity of the reflected interrogation beam power to the photoacoustic signal modulation is at a maximum. The sensitivity of the sensor is proportional to the derivative of the ITF at $\varphi_b$. That is, the optimum bias phase, $\varphi_b$, is selected to be substantially at an extrema (which may be at a maximum) of the derivative of an interferometer transfer function (ITF) for each addressable location.

The selection of the optimum bias phase or, bias wavelength, shall now be described in relation to Figure 4. Figure 4A shows in the unbroken line a representative plot of the Fabry Perot cavity phase difference Interferometer Transfer Function (ITF) of the optical power $P_r$ reflected from the cavity against $\varphi$, the phase difference between the optical fields reflected from the two mirrors of the Fabry Perot interferometer (as controlled by changing the wavelength of the interrogation light source across a range of wavelengths) generated by monitoring the reflected power $P_r$ at the

same addressable location while scanning the interrogation light source 202 across the FSR of the cavity. Note $P_r$ is sometimes denoted the dc level and refers to the reflected power in the absence of the acoustic signal or with the latter filtered out. First, note that the phase difference between the optical fields reflected from the two mirrors of the Fabry Perot interferometer $\varphi$ is given by

$$\varphi = 4\pi n t/\lambda$$

where $n$ is the refractive index, $t$ is the cavity thickness and $\lambda$ is the optical wavelength.

Hence $\varphi$ can be varied by adjusting the wavelength of the interrogation beam $\lambda$, the cavity optical thickness ($nt$), the refractive index n alone, or the physical thickness t. When the acoustic field is indicent on the surface of the Fabry Perot etalon, the incident photoacoustic signal causes the cavity optical thickness, $nt$, to be varied as the cavity reacts to this incident signal. This in turn causes a modulation of the phase difference $\varphi$ of the light in the cavity by an amount $d\varphi$. Due to the variation in the phase difference ITF due to the narrowband response of the Fabry Perot etalon, the phase modulation $d\varphi$ leads to a modulation of the reflected power of an amount $dP_r$.

When operating the phase control means 222 to tune the phase difference to an optimum bias phase, $\varphi_b$ (such as by tuning the wavelength to an optimum bias wavelength) for interrogating an addressable location of the Fabry Perot etalon, the objective is to set the phase modulation amplitude $d\varphi$ between the optical fields reflected by the two mirrors of the cavity such that $\varphi$ corresponds to the point of maximum slope on the phase difference interferometer transfer function ITF. The value of $\varphi$ that corresponds to this point of maximum slope is called the optimum phase bias point $\varphi_b$. That is, the value of $\varphi$ at which the derivative of the phase difference Interferometer Transfer Function, ITF', is at an extrema, in this case the maximum. Under these conditions, the cavity is most sensitive to an acoustically-induced phase shift as the amplitude of the phase modulation $d\varphi$ gives the largest amplitude of the reflected power modulation $dP_r$, giving the highest sensitivity for that location. Further, at $\varphi_b$, the response of the reflected power of the Fabry Perot interferometer to changes in phase is at its most linear.

Given the above relationship between optical phase difference and wavelength, the optimum bias wavelength $\lambda_b$ can be obtained from the optimum phase bias point $\varphi_b$. Alternatively the optimum bias wavelength $\lambda_b$ can be obtained directly by obtaining the wavelength interferometer transfer function (ITF$_\lambda$), evaluating the derivative of the wavelength interferometer transfer function (ITF$_\lambda$') and selecting the laser wavelength corresponding to the maximum of value of ITF$_\lambda$'. This can be seen in relation to Figure 4B, which shows a plot of the wavelength ITF$_\lambda$, the derivative ITF$_\lambda$', and it plots the values of $\lambda_b$ and $P_r(\lambda b)$. $\lambda_0$ corresponds to the minimum value of the ITF$_\lambda$.

In practice, the tuning map for the whole Fabry Perot etalon could be generated by tuning the phase difference of the light in the cavity (e.g. the laser wavelength) over the Free Spectral Range (FSR) of the cavity for each addressable location (x,y) in order to the generate a measured ITF for all addressable locations (x,y), and to identify from this generated ITF a value of the bias phase $\varphi_b$ or wavelength $\lambda_b$ for each of the addressable locations.

However, due to a combination of the relatively slow tuning speed the phase control means 222 (in particular that of suitable interrogation lasers) and the large number of detection points (~10,000) required to reconstruct an accurate 3D photoacoustic image, this characterisation process is too slow for this purpose. Further, due to the slow tuning speed of a suitable laser, holding the laser beam stationary for a relatively long period can result in heating the cavity (self-heating) which can corrupt the ITF measurement resulting in an inaccurate estimation of $\lambda_b$.

Further, selecting the bias phase $\varphi_b$ based on the ITF generated from direct measurements, which are inherently noisy due to various noise sources in the system, results in an unreliable estimate of the ITF derivative which in turn can lead to an inaccurate estimate of $\varphi_b$.

With reference now to Figure 7, in which the tuning map generation process will be described, without limitation, in relation to bias wavelength determination, to implement a method for generating a tuning map disclosed in the present application that is fast and accurate, in use, the sensor tuning module 227 of the controller 221 is configured to, in process activity 710, tuning the wavelength tuneable source to selected characterisation wavelengths ($\lambda_{1...n}$) within the tuneable wavelength range, and for each wavelength, perform the steps 720 and 730.

The sensor tuning module 227 of the controller 221 is also configured to, in process activity 720, at each of the selected characterisation wavelengths ($\lambda_{1...n}$), control the beam directing means to direct the one or more interrogation beams to each of at least a characterisation subset of the addressable locations (x,y) of the sensor head.

The sensor tuning module 227 of the controller 221 is also configured to, in process activity 730, detect, by the detections means, for at least the characterisation subset of the addressable locations (x,y) and each characterisation wavelength ($\lambda_{1...n}$), the power of the one or more reflected interrogation beams, to generate an interferometer transfer function ITF$_G$ (x,y).

In order to characterise the ITF of the Fabry Perot etalon across its surface in a timely and accurate manner, in accordance with the above steps, the interrogation light source 202 is set to a first one of the selected characterisation wavelengths ($\lambda_{1...n}$) and the beam directing means 208 is controlled to perform a rapid x-y scan of all the addressable locations of the sensor. This takes approximately 1 second. At each x,y location, the reflected power $P_r$ is recorded. The wavelength is then incremented by a small amount to the next one of the selected characterisation wavelengths ($\lambda_{1...n}$) and a second x-y scan performed. This process of successively incrementing $\lambda$ and spatially scanning is repeated until a map of the ITFs at each x-y location on the sensor has been generated, referred to as $ITF_G(x,y)=Pr(x,y, \lambda)$. This represents step 1 shown on Figure 5.

Since the interrogation beam is rapidly scanned across the sensor, self-heating at each point is minimal thus avoiding distortion of the $ITF_G$. Furthermore, by tuning the laser in small steps, each separated by the relatively long time required to complete a 2D scan, the laser tuning initialisation and settling times form a much smaller proportion of the total time required to acquire the $ITF_G$ map. This approach is therefore much faster than the naive method of raster scanning at each x-y location at adjusting the phase bias between each.

Further, as, in the following steps, a template ITF, referred to as $ITF_T$, is fitted to the $ITF_G$s for all the addressable locations, the $ITF_G$s may be generated by tuning the interrogation light source 202 to only a subset of all the tuneable wavelengths over the etalon's FSR. This coarse tuning does not diminish the accuracy of the output fitted template ITFs used for assessing the optimum bias wavelength, but rather it avoids further self heating and actually generates an even more accurate, non-distorted output. Further, characterising the Fabry Perot etalon at only a subset of the tuneable wavelengths significantly reduces the time needed to calibrate the sensor head and generate the tuning map. Further, as the natural thickness of the cavity is only relatively slowly varying across the surface of the Fabry Perot etalon, the ITF, and thus the optimum bias wavelength, should not vary significantly for proximal addressable locations on the surface, and so the ITF could be generated for only a subset of all of the addressable locations. In this way, the ITF or the bias wavelength $\lambda_b$ can be interpolated for all addressable locations based on generated $ITF_G$s for a subset of the addressable locations.

To avoid self-heating and obtain the "true" $ITF_G$, the measurement in steps 710-730 is made by operating the interrogation light source 202 at very low power.

The sensor tuning module 227 of the controller 221 is also configured to, in process activity 740, fit a template interferometer transfer function $ITF_T$ to each $ITF_G$ (x,y) to generate a fitted interferometer transfer function $ITF_F(x,y)$ for each of the plurality of addressable locations (x,y).

As indicated above, noise on the measured $ITF_G$ results in an unreliable estimate of the ITF derivative which in turn can lead to an inaccurate estimate of $\lambda_b$. For example, noisy low reflected power and noisy high reflected power measurements at adjacent characterisation wavelengths in an $ITF_G$ can give a maximum value in the $ITF_G$' that does not correspond to the location of the maximum slope of the ITF peak. To address this, a template $ITF_T$, which represents a noise free ITF peak shape for the Fabry Perot etalon, is fitted to the measured $ITF_G$s for each of the addressable locations to generate a fitted $ITF_F$ for each addressable location. The fitted $ITF_F$ is smooth across the FSR wavelengths not coarse and is noise free. From the $ITF_F$s a smooth and accurate derivative ITF' can be estimated.

The template $ITF_T$ may be generated empirically by applying a mathematical operation to the measured $ITF_G$s. However, this seemingly simple task is in fact non trivial. The challenge lies in selecting a suitable function that provides a true representation of the ITF and can be efficiently fitted to the measured $ITF_G$s - computational efficiency is important because of the large number of ITFs that are acquired, as much as one for each scan point (~10,000).

A highly efficient approach involves forming a template $ITF_T$ function from a subset of the measured $ITF_G$s. This template is then rescaled to fit the measured $ITF_G$s at each addressable location. This approach is robust, accurate and computationally very efficient, the latter because it avoids the use of a non-linear fit. The specific details of the methodology are as follows.

The subset of measured $ITF_G$s (~10% of the total number of $ITF_G$s) is obtained by a calculating "quality" metric, typically the visibility, for every $ITF_G$ in the $ITF_G$ map, forming a histogram of that metric and selecting those ITFcs that lie in the top X% of the chosen metric. X is typically 10%. Visibility is defined as:

$$\text{Visibility} = (P_r\text{max} - P_r\text{min}) / (P_r\text{max} + P_r\text{min})$$

where Prmax and Prmin are the maximum and minimum values of the ITF. This is used as the metric for determining the subset because it is robust to noise and can be computed efficiently.

The template $ITF_T$ is obtained as follows. First the minima of the individual $ITF_G$s within the above mentioned subset are aligned in wavelength space. The aligned ITFs are then summed together and normalised to produce a mean ITF. This mean ITF serves as the template ITFT in most cases. However, in some circumstances, the mean ITF is not a sufficiently accurate representation of the true ITF. In this case a model (eg the Lorentzian Airy function) is fitted (using a nonlinear fit) to the mean ITF; since the nonlinear fit is only performed once, the computational cost of this operation is negligible. The fitted function then serves as the $ITF_T$. An example $ITF_T$ is shown in Figure 4B.

$ITF_T$ is then fitted to each measured $ITF_G$ in the map by an appropriate fitting technique. This may include (1) cross-correlating their minima in order to align the $ITF_T$ with the $ITF_G$ in wavelength space and then (2) applying a vertical offset and multiplier (i.e. $a_0 + b(ITF_T)$) in order to fit the template $ITF_T$ to each measured $ITF_G$. Since this is a simple 1-axis scaling operation and avoids using a nonlinear fit, it is efficient and fast.

This fitting process yields in a map of noise-free $ITF_F$s, one for each x,y position on the sensor. This template fitting represents step 2 shown in Figure 5.

The sensor tuning module 227 of the controller 221 is also configured to, in process activity 750, determine, from the fitted interferometer transfer function $ITF_F(x,y)$ for each addressable location (x,y), a respective bias wavelength, $\lambda_b(x,y)$, for the wavelength source at each addressable location (x,y).

In this step, a map of the optimum bias wavelengths $\lambda_b(x, y)$ is formed; this is called the "pre-tuning map" or simply a "tuning map". To form this, the derivative of *only* the $ITF_T$ is computed. As a uniform $ITF_T$ is being used for all x,y, the derivative of each $ITF_F$ does not need to be calculated. From the derivative of the $ITF_T$, the wavelength, $\lambda_b$, corresponding to the maximum value of this derivative is determined. $\Delta\lambda$ (see Figure 4B) is then calculated where $\Delta\lambda$ $=|\lambda b-\lambda 0|$ and $\lambda_0$ is the wavelength corresponding to the minimum value of the $ITF_T$. Since the $ITF_T$ is fitted to the measured $ITF_G$ for each addressable location by aligning and offsetting along the vertical axis only, $\Delta\lambda$ is the same for all fitted ITFs so it is only necessary to compute the derivative for $ITF_T$ not each $ITF_F$. To obtain $\lambda_b(x, y)$ it is only necessary to determine $\lambda_0$ at each x,y position which can be achieved more efficiently than computing the derivative at each point. $\lambda_b$ is then given by $\lambda_b = \lambda_0 \pm \Delta\lambda$. Step 3 of Figure 5 shows an illustrative example of a pre-tuning map acquired using the above method. Each region represents an area with the same $\lambda b$. Physically they represent areas of the same optical thickness.

While the above description sets out tuning map generation by controlling a wavelength of the interrogation light source 202 to determine a tuning map of the bias wavelengths across the Fabry Perot interferometer, it is to be understood that the above method could be applied by controlling for wavelength and/or cavity thickness and/or refractive index to generate a tuning map of comprising biasing values of one or more of the phase control variables.

To compensate for any innate variations in sensitivity across the Fabry Perot etalon, a sensitivity map can be generated. The sensitivity of the sensor is proportional to the derivative of the ITF at $\lambda_b$. Since the $ITF_G$ and its fitted template $ITF_F$ is obtained at each addressable location, a sensitivity map S(x,y) can be formed, for example by evaluating the derivative of the $ITF_F$ at each location. This can be used to correct the detected acoustic signals for spatial variations in sensitivity arising from defects in the sensor, variations in mirror reflectivities and other factors.

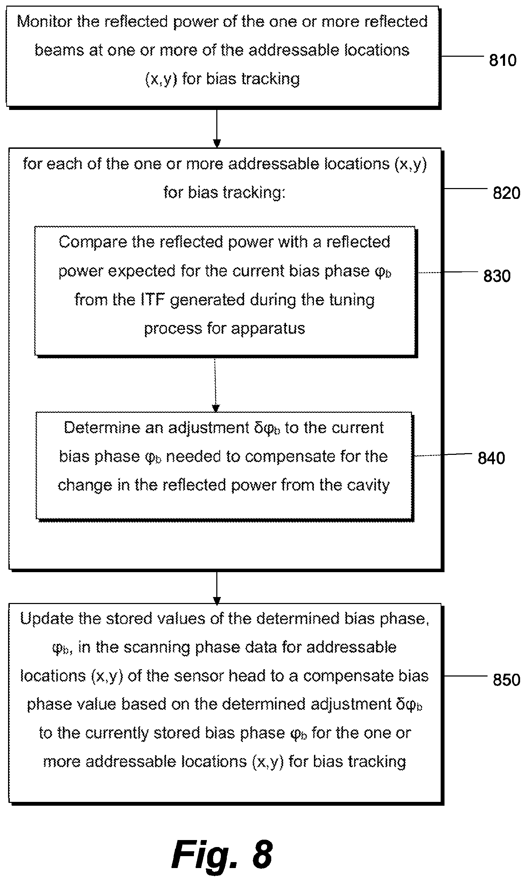

The tuning map can also be compensated for variations in the bias wavelength that occur during use. In this regard, example methods of adjusting the determined bias phase, $\varphi_b$, to compensate for local or bulk changes in the optical path length in the cavity in use, such as due to variations in sensor head temperature and applied pressure since the tuning process, will now be described with reference to Figure 8. The bias phase tracking and adjusting process is described below and with reference to Figure 8 in relation to an imaging ultrasound optical sensor in which the interrogation beam is scanned across addressable locations of the sensor head, and reflected signal power is detected to recover an image from the modulated signal indicative of the acoustic field. However, as is apparent from the present disclosure, the bias tracking method and apparatus disclosed herein can be implemented independent of any need to scan a sensor head for imaging a target. For example, the phase bias tracking method disclosed herein is applicable in a non-imaging ultrasound optical sensor such as a single fibre sensor with a Fabry Perot cavity bonded to the end.

A variety of external influences can cause the pre-tuning map $\lambda_b(x, y)$ to change. For example, if the sensor is placed on the surface of the skin, this will result in the application of a static pressure and a temperature change. Both can cause the cavity thickness to change and thus $\lambda_b$. In some cases (eg a uniform temperature change) the change in $\lambda_b$ is spatially invariant. In others, for example the application of local pressure, it is position dependent. If a temperature or pressure change does occur then the pre-tuning and scan-plan steps described above would need to repeated. However, this would be time consuming which is problematic if a rapid sequence of images is required. In accordance with aspects of the invention, the following compensation methods of single- and multi-point bias wavelength monitoring provide a means of addressing this and rapidly updating the pre-tuning map. Each method involves measuring the reflected power $P_r$ in use and using this to infer if $\lambda_b$ has changed.

Generally in single- and multi-point bias point monitoring, to adjust the determined bias phase $\varphi_b$, (the bias wavelength $\lambda_b$), to compensate for local or bulk changes in the optical path length in the cavity in use, the controller 221 is further configured (for example, either by the interrogation module 225 or the tuning module 227) to, in step 810, in use, monitor the reflected power of the one or more reflected beams at one or more of the addressable locations (x,y) for bias tracking. Then, in step 820, for each of the one or more addressable locations (x,y) for bias tracking, the controller 221 is configured to: in step 830, compare the reflected power with a reflected power expected for the current bias phase $\varphi_b$ from the ITF generated during the tuning process for apparatus; and, in step 840, determine an adjustment $\delta\varphi_b$ to the current bias phase $\varphi_b$ needed to compensate for the change in the reflected power from the cavity. The controller 221 is then configured, in step 850, to update the stored values of the determined bias phase, $\varphi_b$, in the scanning wavelength data 223 for addressable locations (x,y) of the sensor head to a compensate bias phase value based on the determined adjustment $\delta\varphi_b$ to the currently stored bias phase $\varphi_b$ for the one or more addressable locations (x,y) for bias tracking.

In more detail, single point bias wavelength monitoring is the simplest method and involves monitoring $P_r$ at a

single spatial point (at $\lambda_b$) and using this and the previously measured $ITF_G$ (or the fitted $ITF_F$) at that point to estimate $\delta\lambda_b$, the change in $\lambda_b$. It is important to monitor $P_r$ at a wavelength that is equal to $\lambda_b$ or as close as possible to it. At wavelengths far from $\lambda_b$ it is not possible to detect changes in $\lambda_b$ since the slope of the ITF is zero. Once $\delta\lambda_b$ has been determined, the pre-tuning map is then updated as follows:

$$\lambda_b(x, y)' = \lambda_b(x, y) \pm \delta\lambda_b$$

This method assumes that $\delta\lambda_b$ is spatially invariant and the same for all x,y. In some cases, $\delta\lambda_b$ is spatially dependent, e.g. pressing a finger on to the surface of the sensor. Under these circumstances the above single point monitoring method is not optimal.

Spatially varying pressure or temperature changes can be compensated for in multi-point monitoring by scanning the interrogation laser beam over a limited number of points across the sensor in use and recording $P_r$ at each point. Using the previously measured $ITF_G$ (or the fitted $ITF_F$) at each point, a coarse map of $\delta\lambda_b$ can then be obtained. This map is then upsampled and interpolated to match the x,y scan grid in order to obtain a "fully sampled" update of the pre-tuning map, as follows:

$$\lambda_b(x, y)' = \lambda_b(x, y) \pm \delta\lambda_b(x,y)$$

Since this method requires that each spatial point in the coarse map is interrogated at its $\lambda_b$ it is advantageous to choose these points so as to minimise the number of tuning events, ideally, by choosing points over a few regions (or even a single region) of the sensor with the same $\lambda_b$. Since these regions often form lines or curves that extend across a significant proportion of the entire sensor surface, this method can provide a good estimate of spatial variations in cavity thickness over the sensor surface. Regions of common $\lambda_b$ sometimes resemble contours on a map, and so this method may be called contour scanning.

To determine $\delta\lambda_b$ in single- and multi-point bias wavelength tracking, the method used to determine the ITF and track $\lambda_b$ may be different. To implement this method, it is first necessary to measure the $ITF_G$ as described above by sweeping the laser wavelength over the cavity FSR (or some proportion of it) and recording the reflected power $P_r$. However, to avoid self-heating and obtain the "true" ITF, this measurement is made at very low power. This is important since, as indicated above, the degree of self-heating varies as the wavelength is tuned over the ITF reflectance minimum causing the shape of the ITF to be distorted, particularly at high light source power and repeated illumination. Once the "low power" ITF has been acquired, $\lambda_b$ is evaluated, preferably in accordance with Figure 7 described above, and the corresponding reflected power $P_r(\lambda_b)$ is determined.

To detect an acoustic signal in use, the sensor could be operated at $\lambda_b$ at the same low power used to acquire the ITF. However, this would result in low sensitivity. For practical ultrasound detection, the laser power is therefore increased by a factor of M. The corresponding reflected power at $\lambda_b$ should then be $MP_r(\lambda_b)$.

If, in use, the measured reflected power is not equal to $MP_r(\lambda_b)$, then self-heating has occurred and the current laser wavelength no longer corresponds to $\lambda_b$ and needs to be adjusted by some amount, +/- $\delta\lambda_b$.

In the bias tracking method, the wavelength is not iteratively adjusted in order to correctly bias the sensor. Instead, the wavelength shift $\delta\lambda_b$ required to correctly bias the sensor is determined in a single step from the measured low power ITF. This requires scaling the lower power ITF by M in order to estimate what the reflected power $P_r$ corresponding to $\lambda_b$ should be at the higher power used for the acoustic measurement. In other words, this approach to compensating for changes in $\lambda_b$ requires knowledge of the shape of the true ITF (which is obtained from low power ITF) in order to directly estimate $\delta\lambda_b$ and thus identify the correct $\lambda_b$ at different times during use. Once the correct $\lambda_b$ has been determined, Pr is continuously monitored. If it changes, the above procedure is repeated. In this way it is possible to track changes in the bias wavelength and maintain the sensor at optimum sensitivity in the presence of self-heating or external temperature and pressure changes.

The above monitoring methods can be implemented either between successive image acquisitions or during image acquisition, the latter enabling a new scan-plan to be formulated during the acquisition process in preparation for the following acquisition. In this way the optimum bias point is maintained at all positions at all times.

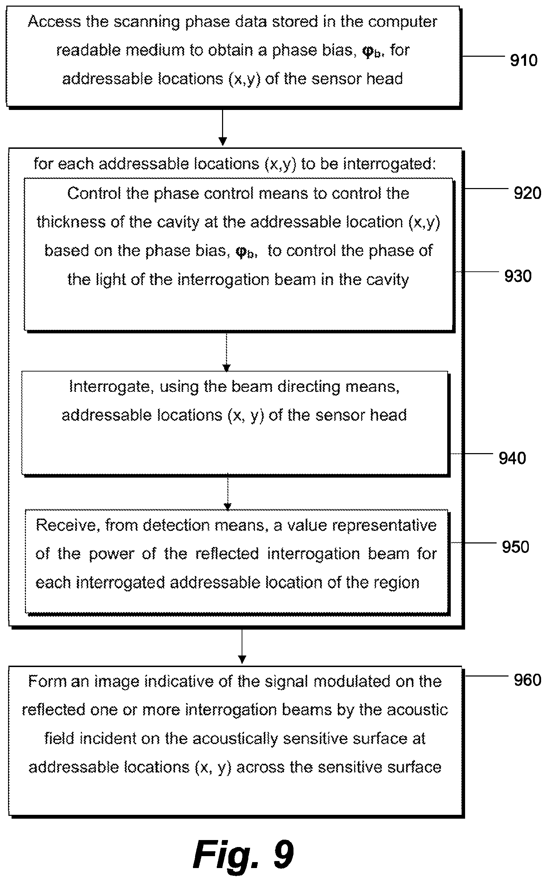

The phase control means 222 may be arranged to control the phase difference of the light of the one or more interrogation beams in the cavity by controlling a thickness of the cavity. an example method for use in performing ultrasound optical imaging with respect to a sample by controlling a thickness of a cavity will now be described with reference to Figure 9.

The method comprises, in step 910, accessing the scanning phase data stored in the computer readable medium to obtain a phase bias, $\varphi_b$, for addressable locations (x,y) of the sensor head. Then, in step 920, for each addressable location (x,y) to be interrogated, the following steps 930 to 950 are performed. In step 930, the phase control means are controlled to control the thickness of the cavity at the addressable location (x,y) based on the phase bias, $\varphi_b$, to control the phase difference of the light of the interrogation beam in the cavity. The phase control means may be configured to control the interrogation light source to generate a heating pulse of controlled energy to heat the sensor head structure and locally heat the cavity to expand the cavity thickness to produce a phase difference of light in the cavity corresponding to the bias phase before interrogating the addressable location to measure the reflected power. The cavity of the sensor head may be configured such that it expands and contracts responsive to operation of the phase control means, and wherein the phase control means is configured to control the cavity to expand or contract the cavity thickness to produce a phase difference of light in the cavity corresponding to the bias phase before interrogating the addressable location to measure the reflected power.

Then, in step 940, addressable locations (x, y) of the sensor head are interrogated using the beam directing means. In step 950, a value representative of the power of the reflected interrogation beam for each interrogated addressable location of the region is received from detection means.

Once the addressable locations have been interrogated, in step 960, an image indicative of the signal modulated on the reflected one or more interrogation beams by the acoustic field incident on the acoustically sensitive surface at addressable locations (x, y) across the sensitive surface is formed, for example using tomographic techniques.

A planar Fabry Perot etalon geometry (as shown in Figure 3A) may be used. For the planar Fabry Perot etalon this is achieved by fabricating a large area etalon and scanning a focused laser beam across in order to address different spatial points.

Alternatively, Fabry Perot etalon may be used having an array of cavity elements have a plano-convex geometry (as shown in Figure 3B). This requires fabricating an etalon structure formed of a 2D array of cavity elements and addressing each individually with a focused laser beam. By providing a sensor head having addressable locations corresponding to plano-convex cavities (as shown in Figure 3B offers significantly higher sensitivity and signal response since it avoids beam walk-off (i.e. the beam is contained at the addressable location), thereby allowing many more round trips within the cavity thus increasing its Q-factor. Other geometries for forming a microresonator arrays as the FP cavity are also possible, such as inverted plano-convex microresonator array.

A single focussed beam of light from the interrogation light source 202 may be used to tune and scan the Fabry Perot sensor head 100. Alternatively, the various components of the interrogation assembly 120 may be configured to tune and/or scan the Fabry Perot sensor head 100 using multiple focussed beams of light. This may be from the interrogation light source 202 generating multiple beams or being split into multiple beams by the beam splitter (e.g. an all-fibre beam splitter), which are then directed appropriately by the beam directing means 208, or from the beam directing means 208 or the wider assembly 120 being configured to split and direct multiple beams from a single beam being emitted from the interrogation light source 202.

In this way, it is possible to scan multiple-beams (typically 8 or 16 beams in a line) to accelerate both the image acquisition process and pre-tuning. This has specific advantages in pre-tuning in particular. Where a single beam source 202 is split between all beams, the wavelength of all beams is identical at all times. Because the variations in the polymer film spacer 106 are small and smoothly varying, it transpires that, over a length of a few mm, the variation in $\lambda_b$ is also small. If all of the beams are located within this length then they will all have a similar $\lambda_b$ and thus provide reasonable sensitivity. Of course, because the spacer 106 thickness is not perfectly uniform, there will still be some variation in $\lambda_b$ and therefore the sensitivity will also vary from one beam to the next to some extent. Image acquisition is clearly faster using multiple beams, but so too is the pre-tuning process. The set of N beams in a multi-beam scanner is referred to as a "block". Typically the block comprises N=8 or 16 beams arranged in a line, although N is arbitrary and other geometries such as a 2D grid can be used.

For tuning using a multi-beam scanner, the single beam process described above in relation to Figure 7 is adapted as follows.

For ITF measurement (steps 710, 720 and 730), this is implemented as described above but using multiple beams. For N beams it will therefore be N times faster.

For ITF fitting (step 740) and formation of the pre-tuning map (step 750), these can also be implemented exactly as described above. Although the measured ITF map is acquired N times faster than with a single beam, the total number of ITFs in the ITF map remains the same so the computation time required to complete steps 740 and 750 is not accelerated by a factor of N. It remains unchanged from the single beam scanner but now forms a much greater proportion of the total pre-tuning time. For this reason, when using a multi-beam system, we typically apply to steps 740 and 750 to the measured $ITF_G$s recorded by only one of the beams within the block. In doing so, a coarse pre-tuning map is generated in a spatial context, and the computation time required to achieve this is reduced by N.

In addition, the computation time required to perform the grouping process and form the scan-plan is also reduced by N. As indicated above, this can be performed as part of the pre-tuning process leading to the generation of the grouped regions or a scan plan for storing in the scanning wavelength data 223, or alternatively the tuning map can be stored and the scan plan generated subsequently, before or on the fly during image acquisition.

In any case, for a multi-beam system the "grouping" process is unchanged and the basic principles that underlie the formation of the "scan-plan" apply but with the following modification.

When scanning the block of beams across the sensor, there will be some scan positions where the block straddles two or more regions of different $\lambda_b$. For example, consider the case when the block is located at a position x,y that spans two regions of different bias wavelengths $\lambda_{b1}$ and $\lambda_{b2}$. When forming the scan-plan, the question then arises as to whether that x,y position should be assigned (or "grouped") to the region having $\lambda_{b1}$ or $\lambda_{b2}$. The assignment may depend on where the majority of beams lie. If only a few of the N beams in the block lie in region 1 then clearly it would be disadvantageous to assign the block located at x,y to $\lambda_{b1}$ in the scan-plan because most of the beams will have a different bias wavelength $\lambda_{b2}$ and therefore provide low sensitivity. Note that this example of just two regions of different $\lambda_b$ is very much a simplification for illustrative purposes.

In reality, $\lambda_b$ does not vary abruptly from one region to the next but smoothly so there will be a gradation of optimum bias wavelengths over the N beams within the block. Hence the general problem can be stated as follows: for a given x,y position of the block, what is the wavelength that will provide the highest sensitivity for the most number of beams in the block.

Whichever wavelength is chosen, it will inevitably be a compromise as not all beams will be at their optimum bias wavelength. The objective is to get the best possible compromise and identify the optimum the wavelength for the block. There are several different approaches that can be used to achieve this compromise.

Fixed beam: The simplest option is to set the wavelength of all beams in the block to the optimum bias wavelength of a specific beam - the master-beam - within the block (eg the central beam). This master-beam can vary depending on x,y position or remain fixed. This is inevitably a compromise but can work reasonably well when the number of different regions of different $\lambda_b$ are small. However, a more sophisticated approach is to make the selection based on some metric that represents the distribution of bias wavelengths or sensitivities across the N beams within the block. While two possible metrics, namely $\lambda_b$ based metrics and Sensitivity based metrics, and approaches to using them are described below, other metrics may also be suitable.

$\lambda_b$ based metrics: the wavelength of the block can be selected on the basis of a metric that is representative of the distribution of $\lambda_b$ over the beams within the block. One method is based on the mean value of $\lambda_b$. Thus, for any given x,y position of the block, $\lambda_b$ is identified for each of the N beams in the block B from the pre-tuning map; $\lambda_{bB1}$, $\lambda_{bB2}$, $\lambda_{bB3}$...... $\lambda_{bBN}$. The average optimum bias wavelength is then calculated

$$\overline{\lambda_{bB}} = \frac{1}{N}\sum_{i=1}^{N}\lambda_{bBi}$$

$\overline{\lambda_{bB}}$ is then taken to be the optimal wavelength for the block when located at x,y. Note that it may be necessary to apply an exclusion criteria to exclude those beams which have extreme bias wavelengths which can otherwise skew the value of $\overline{\lambda_{bB}}$ in such a way that only a few beams are optimally biased or indeed none at all. This is called the "trimmed average method" and computes the mean of $\lambda_{bBi}$ after removing a percentage of potential outliers, typically 5-10% from both the lowest and largest values. The 'mean $\lambda_b$' is a fast method and works effectively when the $\lambda_b$ 'trimmed' histogram range is smaller than $\Delta\lambda$. However, selecting $\lambda_b$ based on metrics other than the mean such as the median or the mode may also be advantageous for specific applications - e.g. the mode might be optimal for deep tissue imaging where the highest absolute sensitivity rather than the mean sensitivity is preferable.

Sensitivity based metrics: one limitation of the using the above $\lambda_b$ based criteria is that the relationship between $\lambda_b$ and sensitivity is somewhat indirect and there may be circumstances where an optimisation based on the distribution of bias wavelengths over the block is non-optimal. A more refined method is to base the wavelength selection on a metric that is directly related to the sensitivity S (the ITF derivative) of each beam for a given x, y position of the block, such as information that is available from the sensitivity map (as outlined above). That is to say, in this approach, S is determined as a function of wavelength for each beam at x,y and then the wavelength that maximises the sum of S over all N beams is selected as $\lambda_b$. A variant of this approach is to maximise $S/\sqrt{P_r}$ which accounts for the shot noise due to the relatively large value of $P_r$.

Implementing the above multi-beam approach has enabled a significant reduction in the time required to complete the entire pre-tuning process - e.g. from 1 minute down to 6 seconds for a 16 beam scanner.

**Claims**

1. Apparatus (200) for performing ultrasound optical imaging with respect to a sample in which an acoustic field is generated in response to a stimulus, the apparatus (200) comprising:

a sensor head (100) having an acoustically sensitive surface arranged as a reflective surface (104a) of a Fabry Perot interferometric cavity, wherein, when the acoustically sensitive surface is placed against the sample, in use, the acoustic field generated in response to said stimulus is incident upon said acoustically sensitive surface to modulate the optical path length in the cavity with a signal indicative of the acoustic field;

a light source (121), optionally wavelength tuneable, for generating one or more interrogation beams of electromagnetic radiation;

beam directing means (208) to direct the one or more interrogation beams (214) onto addressable locations (x,y) across said acoustically sensitive surface;

phase control means (222) for controlling the phase difference between the reflected optical fields of the light of the one or more interrogation beams in the cavity;

detection means (212) configured to receive and determine one or more values representative of the power of the reflected one or more interrogation beams (214) from the addressable locations (x,y);

computer readable medium storing scanning phase data (223) representative of a set of phase values for controlling the phase control means (222) for addressable locations (x,y) of the sensor head (100), the scanning phase data (223) relating:

addressable locations (x,y) of the sensor head (100) onto which the one or more interrogation beams can be directed in use; and

a respective determined bias phase, $\varphi_b$, for the sensor head (100) at given addressable locations (x,y), selected to be a phase difference of the light field in the cavity at which the power of the interrogation beam (214) interferometrically reflected from the Fabry Perot cavity is in use modulated by the signal from the acoustic field incident on the acoustically sensitive surface at that location;

and

a controller (221) configured to:

access the scanning phase data (223) stored in the computer readable medium;

identify from the scanning phase data (223) regions of grouped addressable locations (x,y) having a common bias phase, $\varphi_b$;

for each identified region of the addressable locations (x,y):

control the phase control means (222) to control the phase difference of the light of the interrogation beam in the cavity to the bias phase, $\varphi_b$, of the region;

interrogate, using the beam directing means (208), addressable locations (x, y) of the group of locations making up the region by scanning the one or more interrogation beam (214) to sequentially interrogate each addressable location within the region; and

receive, from detection means (212), values representative of the power of the reflected interrogation beam for each interrogated addressable location of the region; and

form an image indicative of the signal modulated on the reflected one or more interrogation beams by the acoustic field incident on the acoustically sensitive surface at addressable locations (x, y) across the sensitive surface.

2. The apparatus (200) of claim 1 wherein the sensor head (100) is formed as a planar Fabry Perot etalon, wherein the etalon comprises the addressable locations (x,y) of the sensor head onto which the interrogation beam (214) can be directed in use.

3. The apparatus (200) of claim 1 wherein the sensor head (100) is formed to have an array of microresonator Fabry Perot interferometric cavities with plano-convex geometry, wherein individual microresonator cavities correspond to the addressable locations (x,y) of the sensor head (100) onto which the interrogation beam (214) can be directed in use.

4. The apparatus (200) of any preceding claim wherein the determined bias phase, $\varphi_b$, for the addressable locations is selected to be substantially at an extrema of the derivative of an interferometer transfer function (ITF) for each addressable location generated during a tuning process of the apparatus, the ITF characterising the power of the reflected interrogation beam (214) within a Free Spectral Range of the Fabry Perot Interferometer.

5. The apparatus (200) any preceding claim, wherein, to adjust the determined bias phase, $\varphi_b$, to compensate for local or bulk changes in the optical path length in the cavity in use, such as due to variations in sensor head (100) temperature and applied pressure since the tuning process, the controller (221) is further configured to:

in use, monitor the reflected power of the one or more reflected beams at one or more of the addressable locations (x,y) for bias tracking;
for each of the one or more addressable locations (x,y) for bias tracking:

compare the reflected power with a reflected power expected for the current bias phase $\varphi_b$ from the ITF generated during the tuning process for apparatus (200); and
determine an adjustment $\delta\varphi_b$ to the current bias phase $\varphi_b$ needed to compensate for the change in the reflected power from the cavity; and,

update the stored values of the determined bias phase, $\varphi_b$, in the scanning phase data (223) for addressable locations (x,y) of the sensor head (100) to a compensate bias phase value based on the determined adjustment $\delta\varphi_b$ to the currently stored bias phase $\varphi_b$ for the one or more addressable locations (x,y) for bias tracking.

6. Apparatus (200) as claimed in any preceding claim, wherein the beam directing means (208) is an optical fibre or an optical fibre bundle.

7. The apparatus (200) of any preceding claim, wherein the beam directing means (208) is a controllable beam directing means operable to direct the one or more interrogation beams onto addressable locations (x,y) across said acoustically sensitive surface, wherein the controller (221) is configured to control the beam directing means (208) to sequentially interrogate addressable locations (x, y) of the group of locations making up the region, and wherein the controller (208) is optionally further configured to:

interrogate an addressable location at an extremity of the region, and
interrogate further addressable locations within the region, wherein each sequential location is determined as the nearest addressable location to the previously interrogated location.

8. The apparatus (200) of any preceding claim wherein the scanning phase data (221) comprises a pre-tuning map, the pre-tuning map comprising the addressable locations mapped to the respective determined bias phase.

9. The apparatus (200) of any preceding claim wherein:

i) the scanning phase data (221) comprises a look-up table grouping the addressable locations by determined bias phase; and/or
ii) the scanning phase data (221) comprises a scan-plan, the scan-plan comprising an ordered list of addressable locations, wherein addressable locations with the same bias phases are grouped together.

10. The apparatus (200) of any preceding claim further comprising beam splitting means to split the interrogation beam (214) into a plurality of beams.

11. The apparatus (200) of claim 10 wherein the plurality of beams are arranged in a line.

12. The apparatus (200) of any preceding claim, wherein the phase control means (222) is configured to control the phase difference of the light of the one or more interrogation beams in the cavity by one or more of:

• tuning a wavelength of light produced by the wavelength tunable source (121);
• controlling a thickness of the cavity;
• controlling a refractive index of the cavity.

13. A method for ultrasound optical tomography with respect to a sample in which an acoustic field is generated in response to a stimulus, the method for use with an apparatus (200) comprising:

a sensor head (100) having an acoustically sensitive surface arranged as a reflective surface (104a) of a Fabry Perot interferometric cavity, wherein, when the acoustically sensitive surface is placed against the sample, in use, the acoustic field generated in response to said stimulus is incident upon said acoustically sensitive surface to modulate the optical path length in the cavity with a signal indicative of the acoustic field;

a light source (121), optionally wavelength tuneable, for generating one or more interrogation beams of electromagnetic radiation;

controllable beam directing means (208) operable to direct the one or more interrogation beams onto addressable locations (x,y) across said acoustically sensitive surface;

phase control means (222) for controlling the phase difference between the reflected optical fields of the light of the one or more interrogation beams in the cavity;

detection means (212) configured to receive and determine one or more values representative of the power of the reflected one or more interrogation beams from the addressable locations (x,y);

computer readable medium storing scanning phase data (223) representative of a set of phase values for controlling the phase control means (222) for addressable locations (x,y) of the sensor head (100), the scanning phase data (223) relating:

addressable locations (x,y) of the sensor head (100) onto which the one or more interrogation beams can be directed in use; and

a respective determined bias phase, $\varphi_b$, for the sensor head (100) at given addressable locations (x,y), selected to be a phase difference of the light field in the cavity at which the power of the interrogation beam (214) interferometrically reflected from the Fabry Perot cavity is in use modulated by the signal from the acoustic field incident on the acoustically sensitive surface at that location;

the method comprising:

accessing the scanning phase data (223) stored in the computer readable medium;

identifying from the scanning phase data (223) regions of grouped addressable locations (x,y) having a common bias phase, $\varphi_b$;

for each identified region of the addressable locations (x,y):

controlling the phase control means (222) to control the phase difference of the light of the interrogation beam in the cavity to the bias phase, $\varphi_b$, of the region;

interrogating, using the beam directing means (208), addressable locations (x, y) of the group of locations making up the region by scanning the one or more interrogation beam (214) to sequentially interrogate each addressable location within the region; and

receiving, from detection means (212), values representative of the power of the reflected interrogation beam (214) for each interrogated addressable location of the region; and

forming an image indicative of the signal modulated on the reflected one or more interrogation beams by the acoustic field incident on the acoustically sensitive surface at addressable locations (x, y) across the sensitive surface.

**14.** A computer readable medium comprising instructions which, when executed by one or more processors controlling a device according to claim 1, cause the device according to claim 1 to implement the method of claim 13.

**Patentansprüche**

**1.** Vorrichtung (200) zum Durchführen von optischer Ultraschallbildgebung in Bezug auf eine Probe, in der ein akustisches Feld als Reaktion auf einen Stimulus erzeugt wird, wobei die Vorrichtung (200) folgende Elemente umfasst:

einen Sensorkopf (100) mit einer akustisch empfindlichen Oberfläche, die als reflektierende Oberfläche (104a) einer interferometrischen Fabry-Perot-Kavität angeordnet ist, wobei, wenn die akustisch empfindliche Oberfläche gegen die Probe platziert wird, im Gebrauch das akustische Feld, das als Reaktion auf den Stimulus erzeugt wird, auf die akustisch empfindliche Oberfläche einfällt und dabei die optische Weglänge in der Kavität mit einem Signal moduliert, das für das akustische Feld kennzeichnend ist;

eine Lichtquelle (121), optional in der Wellenlänge abstimmbar, zum Erzeugen eines oder mehrerer Abfragestrahlen elektromagnetischer Strahlung;

Strahlrichtmittel (208) zum Richten des einen oder der mehreren Abfragestrahlen (214) auf ansprechbare Stellen (x, y) über die akustisch empfindliche Oberfläche hinweg;

Phasensteuerungsmittel (222) zum Steuern der Phasendifferenz zwischen den reflektierten optischen Feldern des Lichts des einen oder der mehreren Abfragestrahlen in der Kavität;

Erfassungsmittel (212), die dafür ausgelegt sind, einen oder mehrere Werte, die die Leistung des einen oder der mehreren reflektierten Abfragestrahlen (214) von den ansprechbaren Stellen (x, y) repräsentieren, zu empfangen und zu bestimmen;

computerlesbares Medium, das Abtastphasendaten (223) speichert, die einen Satz von Phasenwerten zum Steuern der Phasensteuerungsmittel (222) für ansprechbare Stellen (x, y) des Sensorkopfes (100) repräsentieren, wobei die Abtastphasendaten (223) folgende Elemente in Beziehung setzen:

ansprechbare Stellen (x, y) des Sensorkopfes (100), auf die der eine oder die mehreren Abfragestrahlen im Gebrauch gerichtet werden können; und

eine jeweilige bestimmte Bias-Phase, $\varphi_b$, für den Sensorkopf (100) an gegebenen ansprechbaren Stellen (x, y), die so gewählt wird, dass sie eine Phasendifferenz des Lichtfeldes in der Kavität ist, bei der die Leistung des Abfragestrahls (214), der interferometrisch von der Fabry-Perot-Kavität reflektiert wird, im Gebrauch durch das Signal vom akustischen Feld moduliert wird, das auf die akustisch empfindliche Oberfläche an dieser Stelle einfällt;

und

eine Steuerung (221), die für folgende Vorgänge ausgelegt ist:

Zugreifen auf die im computerlesbaren Medium gespeicherten Abtastphasendaten (223);

Identifizieren, aus den Abtastphasendaten (223), von Bereichen gruppierter ansprechbarer Stellen (x, y) mit einer gemeinsamen Bias-Phase, $\varphi_b$;

für jeden identifizierten Bereich der ansprechbaren Stellen (x, y):

Steuern der Phasensteuerungsmittel (222) zum Steuern der Phasendifferenz des Lichts des Abfragestrahls in der Kavität auf die Bias-Phase, $\varphi_b$, des Bereichs;

Abfragen, unter Verwendung des Strahlrichtmittels (208), von ansprechbaren Stellen (x, y) der Gruppe von Stellen, die den Bereich bilden, durch Abtasten des einen oder der mehreren Abfragestrahlen (214) zum sequentiellen Abfragen jeder ansprechbaren Stelle innerhalb des Bereichs; und

Empfangen, von Erfassungsmitteln (212), von Werten, die für die Leistung des reflektierten Abfragestrahls für jede abgefragte ansprechbare Stelle des Bereichs repräsentativ sind; und

Erzeugen eines Bildes, das für das Signal kennzeichnend ist, das auf den einen oder die mehreren reflektierten Abfragestrahlen durch das akustische Feld moduliert wird, das auf die akustisch empfindliche Oberfläche an ansprechbaren Stellen (x, y) über die empfindliche Oberfläche hinweg einfällt.

2. Vorrichtung (200) nach Anspruch 1, wobei der Sensorkopf (100) als planares Fabry-Perot-Etalon ausgebildet ist, wobei das Etalon die ansprechbaren Stellen (x, y) des Sensorkopfes umfasst, auf die der Abfragestrahl (214) im Gebrauch gerichtet werden kann.

3. Vorrichtung (200) nach Anspruch 1, wobei der Sensorkopf (100) so geformt ist, dass er ein Array von interferometrischen Mikroresonator-Fabry-Perot-Kavitäten mit plankonvexer Geometrie aufweist, wobei einzelne Mikroresonator-Kavitäten den ansprechbaren Stellen (x, y) des Sensorkopfes (100) entsprechen, auf die der Abfragestrahl (214) im Gebrauch gerichtet werden kann.

4. Vorrichtung (200) nach einem der vorhergehenden Ansprüche, wobei die bestimmte Bias-Phase, $\varphi_b$, für die ansprechbaren Stellen so ausgewählt wird, dass sie im Wesentlichen bei einem Extremum der Ableitung einer Interferometer-Übertragungsfunktion, ITF, für jede ansprechbare Stelle liegt, die während eines Abstimmungsprozesses der Vorrichtung erzeugt wird, wobei die ITF die Leistung des reflektierten Abfragestrahls (214) innerhalb eines freien Spektralbereichs des Fabry-Perot-Interferometers charakterisiert.

5. Vorrichtung (200) nach einem der vorhergehenden Ansprüche, wobei die Steuerung (221) zum Einstellen der bestimmten Bias-Phase, $\varphi_b$, zum Kompensieren lokaler oder allgemeiner Änderungen der optischen Weglänge in der Kavität im Gebrauch, wie etwa aufgrund von Variationen der Temperatur des Sensorkopfes (100) und des angelegten Drucks seit dem Abstimmungsprozess, ferner für folgende Vorgänge ausgelegt ist:

im Gebrauch, Überwachen der reflektierten Leistung des einen oder der mehreren reflektierten Strahlen an einem oder mehreren der ansprechbaren Stellen (x, y) zur Bias-Nachführung;

für jede der einen oder der mehreren ansprechbaren Stellen (x, y) zur Bias-Nachführung:

Vergleichen der reflektierten Leistung mit einer reflektierten Leistung, die für die aktuelle Bias-Phase $\varphi_b$ aus der ITF erwartet wird, die während des Abstimmungsprozesses für die Vorrichtung (200) erzeugt wird; und

Bestimmen einer Anpassung $\delta\varphi_b$ für die aktuelle Bias-Phase $\varphi_b$, die erforderlich ist, um die Änderung der von der Kavität reflektierten Leistung zu kompensieren; und,

Aktualisieren der gespeicherten Werte der bestimmten Bias-Phase, $\varphi_b$, in den Abtastphasendaten (223) für ansprechbare Stellen (x, y) des Sensorkopfes (100) auf einen kompensierten Bias-Phasenwert auf der Grundlage der bestimmten Anpassung $\delta\varphi_b$ an die aktuell gespeicherte Bias-Phase $\varphi_b$ für den einen oder die mehreren ansprechbaren Stellen (x, y) zur Bias-Nachführung.

6. Vorrichtung (200) nach einem der vorhergehenden Ansprüche, wobei das Strahlrichtmittel (208) eine optische Faser oder ein optisches Faserbündel ist.

7. Vorrichtung (200) nach einem der vorhergehenden Ansprüche, wobei das Strahlrichtmittel (208) ein steuerbares Strahlrichtmittel ist, das dazu betreibbar ist, den einen oder die mehreren Abfragestrahlen auf ansprechbare Stellen (x, y) über die akustisch empfindliche Oberfläche hinweg zu richten, wobei die Steuerung (221) dafür ausgelegt ist, das Strahlrichtmittel (208) dazu zu steuern, sequentiell ansprechbare Stellen (x, y) der Gruppe von Stellen, die den Bereich bilden, abzufragen, und wobei die Steuerung (208) optional ferner für folgende Vorgänge ausgelegt ist:

Abfragen einer ansprechbaren Stelle an einem Rand des Bereichs, und
Abfragen weiterer ansprechbarer Stellen innerhalb des Bereichs, wobei jede aufeinanderfolgende Stelle als die der zuvor abgefragten Stelle am nächsten liegende ansprechbare Stelle bestimmt wird.

8. Vorrichtung (200) nach einem der vorhergehenden Ansprüche, wobei die Abtastphasendaten (221) eine Vorabstimmungskarte umfassen, wobei die Vorabstimmungskarte die ansprechbaren Stellen umfasst, die der jeweiligen bestimmten Bias-Phase zugeordnet sind.

9. Vorrichtung (200) nach einem der vorhergehenden Ansprüche, wobei:

i) die Abtastphasendaten (221) eine Nachschlagetabelle umfassen, die die ansprechbaren Stellen nach der bestimmten Bias-Phase gruppiert; und/oder
ii) die Abtastphasendaten (221) einen Abtastplan umfassen, wobei der Abtastplan eine geordnete Liste ansprechbarer Stellen umfasst, wobei ansprechbare Stellen mit den gleichen Bias-Phasen zusammen gruppiert werden.

10. Vorrichtung (200) nach einem der vorhergehenden Ansprüche, ferner umfassend Strahlteilungsmittel zum Aufteilen des Abfragestrahls (214) in eine Vielzahl von Strahlen.

11. Vorrichtung (200) nach Anspruch 10, wobei die Vielzahl von Strahlen in einer Linie angeordnet sind.

12. Vorrichtung (200) nach einem der vorhergehenden Ansprüche, wobei die Phasensteuerungsmittel (222) dafür ausgelegt sind, die Phasendifferenz des Lichts des einen oder der mehreren Abfragestrahlen in der Kavität durch einen oder mehrere der folgenden Vorgänge zu steuern:

• Abstimmen einer Wellenlänge des von der in der Wellenlänge abstimmbaren Quelle (121) erzeugten Lichts;
• Steuern einer Dicke der Kavität;
• Steuern eines Brechungsindex der Kavität;

13. Verfahren zur optischen Ultraschalltomographie in Bezug auf eine Probe, in der ein akustisches Feld als Reaktion auf einen Stimulus erzeugt wird, wobei das Verfahren zur Verwendung mit einer Vorrichtung (200) folgende Elemente umfasst:

einen Sensorkopf (100) mit einer akustisch empfindlichen Oberfläche, die als reflektierende Oberfläche (104a) einer interferometrischen Fabry-Perot-Kavität angeordnet ist, wobei, wenn die akustisch empfindliche Oberfläche gegen die Probe platziert wird, im Gebrauch das akustische Feld, das als Reaktion auf den Stimulus erzeugt wird, auf die akustisch empfindliche Oberfläche einfällt und dabei die optische Weglänge in der Kavität mit einem Signal moduliert, das für das akustische Feld kennzeichnend ist;
eine Lichtquelle (121), optional in der Wellenlänge abstimmbar, zum Erzeugen eines oder mehrerer Abfragestrahlen elektromagnetischer Strahlung;

steuerbares Strahlrichtmittel (208), das dazu betreibbar sind, den einen oder die mehreren Abfragestrahlen auf ansprechbare Stellen (x, y) über die akustisch empfindliche Oberfläche hinweg zu richten;

Phasensteuerungsmittel (222) zum Steuern der Phasendifferenz zwischen den reflektierten optischen Feldern des Lichts des einen oder der mehreren Abfragestrahlen in der Kavität;

Erfassungsmittel (212), die dafür ausgelegt sind, einen oder mehrere Werte, die die Leistung des einen oder der mehreren reflektierten Abfragestrahlen von den ansprechbaren Stellen (x, y) repräsentieren, zu empfangen und zu bestimmen;

computerlesbares Medium, das Abtastphasendaten (223) speichert, die einen Satz von Phasenwerten zum Steuern der Phasensteuerungsmittel (222) für ansprechbare Stellen (x, y) des Sensorkopfes (100) repräsentieren,

wobei die Abtastphasendaten (223) folgende Elemente in Beziehung setzen:

ansprechbare Stellen (x, y) des Sensorkopfes (100), auf die der eine oder die mehreren Abfragestrahlen im Gebrauch gerichtet werden können; und

eine jeweilige bestimmte Bias-Phase, $\varphi_b$, für den Sensorkopf (100) an gegebenen ansprechbaren Stellen (x, y), die so gewählt wird, dass sie eine Phasendifferenz des Lichtfeldes in der Kavität ist, bei der die Leistung des Abfragestrahls (214), der interferometrisch von der Fabry-Perot-Kavität reflektiert wird, im Gebrauch durch das Signal vom akustischen Feld moduliert wird, das auf die akustisch empfindliche Oberfläche an dieser Stelle einfällt;

wobei das Verfahren umfasst:

Zugreifen auf die im computerlesbaren Medium gespeicherten Abtastphasendaten (223);

Identifizieren, aus den Abtastphasendaten (223), von Bereichen gruppierter ansprechbarer Stellen (x, y) mit einer gemeinsamen Bias-Phase $\varphi_b$;

für jeden identifizierten Bereich der ansprechbaren Stellen (x, y):

Steuern der Phasensteuerungsmittel (222) zum Steuern der Phasendifferenz des Lichts des Abfragestrahls in der Kavität auf die Bias-Phase, $\varphi_b$, des Bereichs;

Abfragen, unter Verwendung des Strahlrichtmittels (208), von ansprechbaren Stellen (x, y) der Gruppe von Stellen, die den Bereich bilden, durch Abtasten des einen oder der mehreren Abfragestrahlen (214) zum sequentiellen Abfragen jeder ansprechbaren Stelle innerhalb des Bereichs; und

Empfangen, von Erfassungsmitteln (212), von Werten, die für die Leistung des reflektierten Abfragestrahls (214) für jede abgefragte ansprechbare Stelle des Bereichs repräsentativ sind; und Erzeugen eines Bildes, das für das Signal kennzeichnend ist, das auf den einen oder die mehreren reflektierten Abfragestrahlen durch das akustische Feld moduliert wird, das auf die akustisch empfindliche Oberfläche an ansprechbaren Stellen (x, y) über die empfindliche Oberfläche hinweg einfällt.

14. Computerlesbares Medium, umfassend Anweisungen, die bei ihrer Ausführung durch einen oder mehrere Prozessoren, die eine Vorrichtung nach Anspruch 1 steuern, die Vorrichtung nach Anspruch 1 veranlassen, das Verfahren nach Anspruch 13 zu implementieren.

**Revendications**

1. Appareil (200) pour réaliser une imagerie optique ultrasonore par rapport à un échantillon dans lequel un champ acoustique est généré en réponse à un stimulus, l'appareil (200) comprenant :

une tête de capteur (100) comportant une surface acoustiquement sensible agencée comme une surface réfléchissante (104a) d'une cavité interférométrique Fabry-Perot, lorsque la surface acoustiquement sensible est placée contre l'échantillon, en cours d'utilisation, le champ acoustique généré en réponse audit stimulus étant incident sur ladite surface acoustiquement sensible pour moduler la longueur du chemin optique dans la cavité avec un signal indicatif du champ acoustique ;

une source lumineuse (121), éventuellement réglable en longueur d'onde, pour générer un ou plusieurs faisceaux d'interrogation de rayonnement électromagnétique ;

un moyen de direction de faisceau (208) pour diriger le ou les faisceaux d'interrogation (214) sur des emplacements adressables (x,y) à travers ladite surface acoustiquement sensible ;

un moyen de commande de phase (222) pour commander la différence de phase entre les champs optiques réfléchis de la lumière du ou des faisceaux d'interrogation dans la cavité ;

un moyen de détection (212) configuré pour recevoir et déterminer une ou plusieurs valeurs représentatives de la puissance du ou des faisceaux d'interrogation réfléchis (214) à partir des emplacements adressables (x,y) ;

un support lisible par ordinateur stockant des données de phase de balayage (223) représentatives d'un ensemble de valeurs de phase pour commander le moyen de commande de phase (222) pour des emplacements adressables (x,y) de la tête de capteur (100), les données de phase de balayage (223) concernant :

des emplacements adressables (x,y) de la tête de capteur (100) sur lesquels le ou les faisceaux d'interrogation peuvent être dirigés en cours d'utilisation ; et

une phase de polarisation déterminée respective, $\varphi_b$, pour la tête de capteur (100) à des emplacements adressables donnés (x,y), sélectionnée pour être une différence de phase du champ lumineux dans la cavité à laquelle la puissance du faisceau d'interrogation (214) réfléchi par interférométrie à partir de la cavité Fabry-Perot est modulée en cours d'utilisation par le signal du champ acoustique incident sur la surface acoustiquement sensible à cet emplacement ; et

un dispositif de commande (221) configuré pour

accéder aux données de phase de balayage (223) stockées sur le support lisible par ordinateur ;

identifier, à partir des données de phase de balayage (223), des régions d'emplacements adressables groupés (x,y) ayant une phase de polarisation commune, $\varphi_b$ ;

pour chaque région identifiée des emplacements adressables (x,y) :

commander le moyen de commande de phase (222) pour commander la différence de phase de la lumière du faisceau d'interrogation dans la cavité par rapport à la phase de polarisation, $\varphi_b$, de la région ;

interroger, à l'aide du moyen de direction de faisceau (208), des emplacements adressables (x, y) du groupe d'emplacements constituant la région en balayant le ou les faisceaux d'interrogation (214) pour interroger séquentiellement chaque emplacement adressable dans la région ; et

recevoir, à partir du moyen de détection (212), des valeurs représentatives de la puissance du faisceau d'interrogation réfléchi pour chaque emplacement interrogé de la région ; et

former une image indiquant le signal modulé sur le ou les faisceaux d'interrogation réfléchis par le champ acoustique incident sur la surface acoustiquement sensible à des emplacements adressables (x, y) sur la surface sensible.

2. Appareil (200) selon la revendication 1, la tête de capteur (100) étant formée comme un étalon de Fabry-Perot planaire, l'étalon comprenant les emplacements adressables (x, y) de la tête de capteur sur lesquels le faisceau d'interrogation (214) peut être dirigé en cours d'utilisation.

3. Appareil (200) selon la revendication 1, la tête de capteur (100) étant formée pour avoir un réseau de cavités interférométriques Fabry-Perot de microrésonateur avec une géométrie plano-convexe, les cavités de microrésonateur individuelles correspondant aux emplacements adressables (x,y) de la tête de capteur (100) sur lesquels le faisceau d'interrogation (214) peut être dirigé en cours d'utilisation.

4. Appareil (200) selon n'importe quelle revendication précédente, la phase de polarisation déterminée, $\varphi_b$, pour les emplacements adressables étant sélectionnée pour être sensiblement à des extremums de la dérivée d'une fonction de transfert d'interféromètre (ITF) pour chaque emplacement adressable généré pendant un processus de réglage de l'appareil, l'ITF caractérisant la puissance du faisceau d'interrogation réfléchi (214) dans une plage spectrale libre de l'interféromètre de Fabry-Perot.

5. Appareil (200) selon n'importe quelle revendication précédente, pour ajuster la phase de polarisation déterminée, $\varphi_b$, afin de compenser les changements locaux ou globaux dans la longueur du chemin optique dans la cavité en cours d'utilisation, tels que ceux dus aux variations de la température de la tête de capteur (100) et de la pression appliquée depuis le processus de réglage, le dispositif de commande (221) étant en outre configuré pour :

en cours d'utilisation, surveiller la puissance réfléchie du ou des faisceaux réfléchis à un ou plusieurs des emplacements adressables (x,y) pour le suivi de polarisation ;

pour chacun du ou des emplacements adressables (x,y) pour le suivi de polarisation :

comparer la puissance réfléchie à une puissance réfléchie attendue pour la phase de polarisation actuelle $\varphi_b$ à partir de l'ITF généré pendant le processus de réglage de l'appareil (200) ; et
déterminer un ajustement $\delta\varphi_b$ de la phase de polarisation actuelle $\varphi_b$ nécessaire pour compenser le changement de la puissance réfléchie par la cavité ; et,

mettre à jour les valeurs stockées de la phase de polarisation déterminée, $\varphi_b$, dans les données de phase de balayage (223) pour des emplacements adressables (x, y) de la tête de capteur (100) à une valeur de phase de polarisation de compensation basée sur l'ajustement déterminé $\delta\varphi_b$ à la phase de polarisation actuellement stockée $\varphi_b$ pour le ou les emplacements adressables (x, y) pour le suivi de polarisation.

6. Appareil (200) selon l'une quelconque des revendications précédentes, le moyen de direction de faisceau (208) étant une fibre optique ou un faisceau de fibres optiques.

7. Appareil (200) selon n'importe quelle revendication précédente, le moyen de direction de faisceau (208) étant un moyen de direction de faisceau commandable opérationnel pour diriger le ou les faisceaux d'interrogation sur des emplacements adressables (x, y) à travers ladite surface acoustiquement sensible, le dispositif de commande (221) étant configuré pour commander le moyen de direction de faisceau (208) pour interroger séquentiellement des emplacements adressables (x, y) du groupe d'emplacements constituant la région, et le dispositif de commande (208) étant éventuellement configuré en outre pour :

interroger un emplacement adressable à une extrémité de la région, et
interroger d'autres emplacements adressables dans la région, chaque emplacement séquentiel étant déterminé comme étant l'emplacement adressable le plus proche de l'emplacement interrogé précédemment.

8. Appareil (200) selon n'importe quelle revendication précédente, les données de phase de balayage (221) comprenant une carte de préréglage, la carte de préréglage comprenant les emplacements adressables mis en correspondance avec la phase de polarisation déterminée respective.

9. Appareil (200) selon n'importe quelle revendication précédente,

i) les données de phase de balayage (221) comprenant une table de recherche regroupant les emplacements adressables par phase de polarisation déterminée ; et/ou
ii) les données de phase de balayage (221) comprenant un plan de balayage, le plan de balayage comprenant une liste ordonnée d'emplacements adressables, les emplacements adressables ayant les mêmes phases de polarisation étant regroupés ensemble.

10. Appareil (200) selon n'importe quelle revendication précédente comprenant en outre un moyen de division de faisceau pour diviser le faisceau d'interrogation (214) en une pluralité de faisceaux.

11. Appareil (200) selon la revendication 10, la pluralité de faisceaux étant agencée en ligne.

12. Appareil (200) selon n'importe quelle revendication précédente, le moyen de commande de phase (222) étant configuré pour commander la différence de phase de la lumière du ou des faisceaux d'interrogation dans la cavité par un ou plusieurs des moyens suivants :

• en réglant une longueur d'onde de la lumière produite par la source réglable en longueur d'onde (121) ;
• en commandant une épaisseur de la cavité ;
• en commandant un indice de réfraction de la cavité.

13. Procédé de tomographie optique par ultrasons par rapport à un échantillon dans lequel un champ acoustique est généré en réponse à un stimulus, le procédé étant destiné à être utilisé avec un appareil (200) comprenant :

une tête de capteur (100) ayant une surface acoustiquement sensible agencée comme une surface réfléchissante (104a) d'une cavité interférométrique Fabry-Perot, lorsque la surface acoustiquement sensible est placée contre l'échantillon, en cours d'utilisation, le champ acoustique généré en réponse audit stimulus étant incident sur ladite surface acoustiquement sensible pour moduler la longueur du chemin optique dans la cavité avec un signal indicatif du champ acoustique ;

une source lumineuse (121), éventuellement réglable en longueur d'onde, pour générer un ou plusieurs faisceaux d'interrogation de rayonnement électromagnétique ;

un moyen de direction de faisceau commandable (208) permettant de diriger le ou les faisceaux d'interrogation sur des emplacements adressables (x,y) à travers ladite surface acoustiquement sensible ;

un moyen de commande de phase (222) pour commander la différence de phase entre les champs optiques réfléchis de la lumière du ou des faisceaux d'interrogation dans la cavité ;

un moyen de détection (212) configuré pour recevoir et déterminer une ou plusieurs valeurs représentatives de la puissance du ou des faisceaux d'interrogation réfléchis à partir des emplacements adressables (x,y) ;

un support lisible par ordinateur stockant des données de phase de balayage (223) représentatives d'un ensemble de valeurs de phase pour commander le moyen de commande de phase (222) pour les emplacements adressables (x,y) de la tête de capteur (100), les données de phase de balayage (223) concernant :

des emplacements adressables (x,y) de la tête de capteur (100) sur lesquels le ou les faisceaux d'interrogation peuvent être dirigés en cours d'utilisation ; et

une phase de polarisation déterminée respective, $\varphi_b$, pour la tête de capteur (100) à des emplacements adressables donnés (x,y), sélectionnée pour être une différence de phase du champ lumineux dans la cavité à laquelle la puissance du faisceau d'interrogation (214) réfléchi par interférométrie à partir de la cavité Fabry-Perot est modulée en cours d'utilisation par le signal du champ acoustique incident sur la surface acoustiquement sensible à cet emplacement ;

le procédé comprenant :

l'accès aux données de phase de balayage (223) stockées sur le support lisible par ordinateur ;

l'identification, à partir des données de phase de balayage (223), de régions d'emplacements adressables groupés (x,y) ayant une phase de polarisation commune, $\varphi_b$ ;

pour chaque région identifiée des emplacements adressables (x,y) :

la commande du moyen de commande de phase (222) pour commander la différence de phase de la lumière du faisceau d'interrogation dans la cavité par rapport à la phase de polarisation, $\varphi_b$, de la région ;

l'interrogation, à l'aide du moyen de direction de faisceau (208), d'emplacements adressables (x, y) du groupe d'emplacements constituant la région en balayant le ou les faisceaux d'interrogation (214) pour interroger séquentiellement chaque emplacement adressable dans la région ; et

la réception, à partir du moyen de détection (212), de valeurs représentatives de la puissance du faisceau d'interrogation réfléchi (214) pour chaque emplacement adressable interrogé de la région ; et

la formation d'une image indiquant le signal modulé sur le ou les faisceaux d'interrogation réfléchis par le champ acoustique incident sur la surface acoustiquement sensible à des emplacements adressables (x, y) sur la surface sensible.

14. Support lisible par ordinateur comprenant des instructions qui, lorsqu'elles sont exécutées par un ou plusieurs processeurs commandant un dispositif selon la revendication 1, amènent le dispositif selon la revendication 1 à mettre en œuvre le procédé selon la revendication 13.

**FIG. 1**

FIG. 2

# FIG. 3A

# FIG. 3B

## FIG. 4A

## FIG. 4B

FIG. 5

Accessing the scanning wavelength data stored in the computer readable medium ——610

Identifying from the scanning wavelength data regions of grouped addressable locations (x,y) having a common bias wavelength $\lambda_b$ ——620

For each identified region of the addressable locations (x,y) ——630

Tuning the wavelength tuneable source to the bias wavelength, $\lambda_b$, of the region ——632

Controlling the beam directing means to sequentially interrogate addressable locations (x, y) of the group of locations making up the region ——634

Receiving, from detection means, values representative of the power of the reflected interrogation beam for each interrogated addressable location of the region ——636

Forming an image indicative of the signal modulated on the reflected one or more interrogation beams by the acoustic field incident on the acoustically sensitive surface at addressable locations (x, y) across the sensitive surface ——640

# FIG. 6

Tuning the wavelength tuneable source to selected characterisation wavelengths ($\lambda_{1...n}$) within the tuneable wavelength range —710

At each wavelength, controlling the beam directing means to direct the one or more interrogation beams to each of at least a characterisation subset of the addressable locations (x,y) of the sensor head —720

Detecting, by the detections means, for at least the characterisation subset of the addressable locations (x,y) and each characterisation wavelength ($\lambda_{1...n}$), the power of the one or more reflected interrogation beams, to generate an interferometric transfer function $ITF_G$ (x,y) —730

Fitting a template interferometric transfer function $ITF_T$ to each $ITF_G$ (x,y) to generate a fitted interferometric transfer function $ITF_F$ (x,y) for each of the plurality of addressable locations (x,y) —740

Determining, from the fitted interferometric transfer function $ITF_F$ (x,y) for each addressable location (x,y), a respective bias wavelength, $_b$( x,y), for the wavelength source at each addressable location (x,y), selected to be a tuned wavelength of the source at which the power of the interrogation beam interferometrically reflected from the Fabry Perot cavity is in use modulated by the signal from the acoustic field incident on the acoustically sensitive surface at that location —750

# FIG. 7

Monitor the reflected power of the one or more reflected beams at one or more of the addressable locations (x,y) for bias tracking ——— 810

for each of the one or more addressable locations (x,y) for bias tracking: ——— 820

Compare the reflected power with a reflected power expected for the current bias phase $\varphi_b$ from the ITF generated during the tuning process for apparatus ---- 830

Determine an adjustment $\delta\varphi_b$ to the current bias phase $\varphi_b$ needed to compensate for the change in the reflected power from the cavity ---- 840

Update the stored values of the determined bias phase, $\varphi_b$, in the scanning phase data for addressable locations (x,y) of the sensor head to a compensate bias phase value based on the determined adjustment $\delta\varphi_b$ to the currently stored bias phase $\varphi_b$ for the one or more addressable locations (x,y) for bias tracking ——— 850

# Fig. 8

Access the scanning phase data stored in the computer readable medium to obtain a phase bias, $\varphi_b$, for addressable locations (x,y) of the sensor head — 910

for each addressable locations (x,y) to be interrogated: — 920

Control the phase control means to control the thickness of the cavity at the addressable location (x,y) based on the phase bias, $\varphi_b$, to control the phase of the light of the interrogation beam in the cavity — 930

Interrogate, using the beam directing means, addressable locations (x, y) of the sensor head — 940

Receive, from detection means, a value representative of the power of the reflected interrogation beam for each interrogated addressable location of the region — 950

Form an image indicative of the signal modulated on the reflected one or more interrogation beams by the acoustic field incident on the acoustically sensitive surface at addressable locations (x, y) across the sensitive surface — 960

*Fig. 9*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2016095520 A1

**Non-patent literature cited in the description**

- **EDWARD ZHANG.** Backward-mode multiwavelength photoacoustic scanner using a planar Fabry-Perot polymer film ultrasound sensor for high-resolution three-dimensional imaging of biological tissues. *Applied Optics,* 01 February 2008, vol. 47, 561